# EUROPEAN PATENT SPECIFICATION

(11) **EP 2 769 220 B1**
(45) Date of publication and mention of the grant of the patent: **15.07.2020**
(21) Application number: 12772445.8
(22) Date of filing: 14.08.2012
(51) Int. Cl.: G01N 33/564, A61K 38/17, C07K 14/47, G01N 33/538, C07K 16/28

(54) **Biomarker for autoimmune disease**
Biomarker für autoimmunologische Krankheit
Biomerqueur pour la maladie autoimmunologique

(30) Priority: 17.10.2011 GR 20110100593
(43) Date of publication of application: 27.08.2014
(73) Proprietor: Tzartos, Socrates, 116 21 Kaisariani (GR)
(72) Inventor: TZARTOS, Ioannis, 116 21 Kaisariani (GR); STERGIOU, Christos, 131 23 Ilion (GR); KILINDIREAS, Konstantinos, 174 55 Kalamiki (GR)
(74) Representative: D Young & Co LLP
(86) International application number: PCT/IB2012/054142
(87) International publication number: WO 2013/057599

(56) References cited:
- US-A- 5 741 671
- Nikolov, NP ET AL.: "Autoantibodies against aquaporins in primary Sjögren's Syndrome (pSS)", , 1 January 2008 (2008-01-01), XP002679357, Retrieved from the Internet: URL:https://acr.confex.com/acr/2008/webpro gram/Paper3359.html [retrieved on 2012-07-05]
- SMITH B L ET AL: "Human red cell aquaporin CHIP. I. Molecular characterization of ABH and Colton blood group antigens.", THE JOURNAL OF CLINICAL INVESTIGATION SEP 1994 LNKD- PUBMED:7521882, vol. 94, no. 3, September 1994 (1994-09), pages 1043-1049, XP055031929, ISSN: 0021-9738
- DENKER B M ET AL: "Identification, purification, and partial characterization of a novel Mr 28,000 integral membrane protein from erythrocytes and renal tubules.", THE JOURNAL OF BIOLOGICAL CHEMISTRY 25 OCT 1988 LNKD- PUBMED:3049610, vol. 263, no. 30, 25 October 1988 (1988-10-25), pages 15634-15642, XP002679358, ISSN: 0021-9258
- ELEONORA FOGLIO ET AL: "Aquaporins and Neurodegenerative Diseases", CURRENT NEUROPHARMACOLOGY, vol. 8, no. 2, 1 June 2010 (2010-06-01), pages 112-121, XP055031684, ISSN: 1570-159X, DOI: 10.2174/157015910791233150
- JOHN S. TZARTOS ET AL: "Anti-Aquaporin-1 Autoantibodies in Patients with Neuromyelitis Optica Spectrum Disorders", PLOS ONE, vol. 8, no. 9, 23 September 2013 (2013-09-23), page e74773, XP55191149, DOI: 10.1371/journal.pone.0074773

## Description

### Field of the Invention

This invention relates to a novel autoantibody marker against aquaporin-1 (AQP1), and methods of diagnosing and treating autoimmune diseases associated with the formation of demyelinated lesions of the central nervous system.

### Background of the invention

The use of biomarkers in clinical diagnostics and drug development is becoming increasingly important. However, the identification of relevant biomarkers often represents a significant challenge. For example, in most instances, no specific biomarkers for lesions or mechanisms of lesion formation in the central nervous system (CNS) are known, with viruses or autoimmunity against unknown antigens being proposed.

Aquaporins are a group of channel proteins, important for the rapid transport, primarily of water, in response to osmosis (Pasantes-Morales and Cruz-Rangel (2010) Neuroscience 168(4): 871-884). These proteins play a role in water balance in the CNS crucial for the integrity of the CNS parenchyma (Amiry-Moghaddam and Ottersen (2003) Nat Rev Neurosci 4(12): 991-1001; Lukaszewicz et al. (2011) Curr Opin Anaesthesiol 24(2): 138-143).

Aquaporin 4 (AQP4) and aquaporin 1 (AQP1) are the main aquaporins which have been detected in the brain (Badaut et al. (2002) J Cereb Blood Flow Metab 22(4): 367-378; Satoh et al. (2007) Neuropathology 27(3): 245-256). In mice, the absence of AQP4 prevents cytotoxic edema during ischemia (Manley et al. (2000) Nat Med 6(2): 159-163; Amiry-Moghaddam and Ottersen (2003) Nat Rev Neurosci 4(12): 991-1001), but inhibits the resolution of vasogenic edema (Papadopoulos et al. (2004) FASEB J 18(11): 1291-1293).

AQP1 in mice is not expressed in astrocytes but is detected specifically in the epithelium of the choroid plexus and in some neurons and may be involved in cerebrospinal fluid (CSF) formation without a direct role in brain edema. In an ischemic mouse brain model, no effect of AQP1 levels or cell types expressing AQP1 was detected during brain ischemia (Ribeiro Mde et al. (2006) J Neurosci Res 83(7): 1231-1240).

In addition to the CNS, AQP1 has been also detected in the peripheral nervous system (Ma, T. H., H. W. Gao, et al. (2011) Acta Pharmacol Sin 32(6): 711-715) and in non-neuronal tissues like red blood cells, liver, kidney, lungs, and sweat glands.

The presence of autoantibodies against mouse astrocytic end feet has been selectively detected in sera from patients with demyelinated lesions in the CNS, usually with the specific characteristics of neuromyelitis optica, NMO (Lennon et al. (2004) Lancet 364(9451): 2106-2112). NMO is a chronic inflammatory demyelinating disease of the CNS, mainly affecting the optic nerves and the spinal cord. Like multiple sclerosis (MS), NMO usually follows a relapsing-remitting course, but it often leads to more severe disability with impairment of functional vision and/or loss of ambulation. Its differentiation from MS and other similar CNS diseases is important in order to apply the appropriate treatment. However, such differentiation is usually very difficult due to overlapping clinical presentation and magnetic resonance imaging (MRI) findings.

The autoantibodies against astrocytic end feet have been called NMO-IgG. Furthermore, it was shown that NMO-IgG bind selectively to the AQP4 water channel (Lennon et al. (2005) J Exp Med 202(4): 473-477), linking NMO with regional loss of AQP4 from astrocytes (Kale et al. (2009) Arch Neurol 66(10): 1298-1299). In vitro, the pathogenic role of anti-AQP4 antibodies has been shown, with accumulations of glutamate, possibly causing a pathogenic environment to oligodendrocytes and neurons (Hinson et al. J Exp Med 205(11): 2473-2481).

Several other AQPs have been detected in various regions and cell types of mammalian CNS (mostly studied in rodents) including astrocytes, oligodendrocytes, neurons and ependymal cells.More specifically,
a. Aquaporin-2 (AQP2) has been detected in several regions of the CNS including the ependymal cell layer, spinal cord, subcortical white matter and hippocampus (Mobasheri et al., 2005, J. Mol. Histol. 36, 1-14).
b. Aquaporin-5 (AQP5) has been detected in astrocytes and neurons in culture (Yamamoto et al., 2001, Brain Res. Mol. Brain Res. 90, 26-38). Its expression showed a transient up-regulation and subsequent down-regulation within 20 h of reoxygenation after hypoxia. This suggests that AQP5 may contribute to the induction of the intracranial edema in the CNS after ischemia injury (Albertini and Bianchi, 2010, Curr. Neuropharmacol. 2010 (8), 84-91).
c. Aquaporin-7 (AQP7) was found in choroid plexus, ependyma, pia and blood vessels of the mouse brain during perinatal development (Shin et al. 2006, Neurosci. Lett. 409, 106-111).
d. Aquaporin-8 (AQP8) expression was found in the spinal cord, especially in the ependymal cells lining its central canal, and possibly In astrocytes (Oshio et al. 2004, Neuroscience, 127, 685-693). Based on these findings It was suggested that AQP8 may facilitate water transport into the central canal of the spinal cord (Albertini and Bianchi, 2010, Curr. Neuropharmacol. 2010. (8), 84-91).

In addition to their presence and function in the brain, these AQPs are also present in other organs and tissues of the body (reviewed in Verkman 2011, J Cell Sci. 2011 124:2107-12; Verkman 2012 Annu Rev Med. 63:303-16). Specifically,
AQP2 is the only AQP regulated by vasopressin expressed in kidney cells for reabsorption of water. In humans, genetic mutations in AQP2 result in nephrogenic diabetes insipidus, characterized by polyuria and urinary hypo-osmolality.

AQP5, is expressed in the kidney, salivary and airway submucosal glands, show defective secretion of saliva and airway mucus and tears.

AQP7 is expressed in adipocytes and facilitates glycerol in order to prevent fat accumulation. AQP8 is expressed in organs of the mammalian gastrointestinal tract (salivary gland, liver, pancreas, small Intestine, and colon) and in the testes, heart, kidney, and airways.

US 5741671A discloses that AQP1 or AQP5 may be used in immunoassay procedures to detect autoantibodies as a diagnostic tool for assisting in identification of individuals suffering from autoimmune diseases which involve immune attack on specific water channel proteins e.g., Sjogren syndrome.

Nikolov et al. (American College of Rheumatology, 2008 Annual Scientific Meeting (24-29 Oct); Presentation: Autoantibodies against aquaporins in primary Sjogren's Syndrome (pSS)) shows that autoantibodies against AQP1 and AQP5 do not contribute to primary Sjogren's Syndrome.

Smith et al. (J. Clin. Invest. 94,1043-1049, 1994) discloses the molecular characterization of ABH and Colton blood group antigens.

Denker et al. (JBC 263, 15634-15642, 1998) discloses the identification, purification, and partial characterization of an integral membrane protein from erythrocytes and renal tubules.

Foglio et al. (Curr. Neuropharmacol. 8, 112-121, 2010) hypothesizes that aquaporins may be abnormally expressed in some pathological conditions such as neurodegenerative diseases.

The present invention identifies anti-AQP1, aquaporin-2 (AQF2), aquaporin-5 (AQP5), aquaporin-7 (AQP7) and aquaporin-8 (AQP8) antibodies as a novel biomarkers for disease.

### Summary of the invention

According to a first aspect of the invention there is provided a method of diagnosis or prognosis of an autoimmune disease associated with the formation of demyelinated lesions of the central nervous system (CNS) in a subject wherein the method comprises detecting aquaporin-1 (AQP1) autoantibodies in a sample obtained from said subject. The method according to this aspect of the invention may further comprise detecting aquaporin-4 (AQP4) autoantibodies in the same or a different sample obtained from said subject.

The level of AQP1 autoantibodies may be compared to the corresponding level of AQP1 autoantibodies in a sample from a healthy subject. By a healthy subject it is meant a corresponding subject that does not have an autoimmune disease characterised by AQP1 autoantibody expression.

According to a second aspect of the present invention there is provided a method of monitoring the progress of an autoimmune disease associated with the formation of demyelinated lesions of the CNS in a subject comprising the steps of:
(i) providing a first sample from the subject,
(ii) detecting AQP1 autoantibodies in said first sample,
(iii) providing a second sample from the subject wherein said second sample is obtained from the subject after said first sample,
(iv) comparing the level of the AQP1 autoantibodies in the second sample with the corresponding level of AQP1 autoantibodies in the first sample, wherein an increase in level of the AQP1 autoantibodies in the second sample relative to the first sample indicates disease progression.

An increase in AQP1 autoantibody expression in the second sample relative to the first sample may indicate disease progression. A decrease in AQP1 autoantibody expression in the second sample relative to the first sample may indicate disease regression.

The method according to this aspect of the invention may also involve detecting AQP4 autoantibodies in said first and second or subsequent samples.

The AQP4 autoantibodies may be detected in the same or different first sample obtained from said subject and the same or different second or subsequent sample obtained from said subject.

Preferably the second sample is obtained from the subject at least 1, 2, 5, 10, 20, 50, 100, 200 or 300 days after the first sample.

The second aspect of the invention may be used to monitor the progress of an autoimmune disease in response to treatment e.g. immunomodulatory therapy (immunosuppression, plasmapheresis, Ig-apheresis, IVIG, therapeutic administration of AQP1 in various forms including in liposomes or nanoparticles, or by nasal or oral administration, T-cell therapy etc). Thus, the present invention may be used to assess the effectiveness of a treatment. The treatment may be administered between the taking of the first sample and the taking of the second or a subsequent sample from the patient.

The AQP1 autoantibodies may be detected by contacting said sample with the respective AQP1 protein, or a cell or tissue comprising said polypeptide, and detecting the presence or absence of binding of said AQP1 autoantibodies to said polypeptide, cell or tissue.

Thus, the AQP1 protein, or cell or tissue comprising the same is used to bind AQP1 autoantibodies.

There is also described herein a method of detecting the presence or absence of AQP1 autoantibodies in a sample obtained from a subject comprising contacting said sample with the respective AQP1 protein, or a cell or tissue comprising said polypeptide, and detecting the presence or absence of binding of said AQP1 autoantibodies to said polypeptide, cell or tissue. (e.g. human embryonic kidney (HEK) cells. mouse brain sections, liver sections or any other AQP1 -containing tissue).

The sample used in the present invention may be, but is not limited to, tissue biopsy, nervous tissue, blood, blood cells, blood plasma, blood serum, blood immunoglobulins or cerebrospinal fluid.

Preferably the detection of AQP1 autoantibodies described herein is achieved using an immunoassay such as an enzyme-linked immunosorbent assay (ELISA), radioimmunoassay (RIA), immunoprecipitation assay, immunofluorescence assay (IFA), indirect immunofluorescence assay (IIF), enzyme linked assay (EIA), luminescence immunoassay (LIA), Western Blot assay (WB) or cell based assay (CBA).

According to another aspect of the present invention there is provided an AQP1 polypeptide, or a cell or tissue comprising said polypeptide, for use in diagnosing an autoimmune disease associated with the formation of demyelinated lesions of the CNS.

The autoimmune disease referred to herein is a disease associated with the formation of demyelinated lesions of the CNS. Demyelination is destruction/loss of myelin sheath from a myelinated area of the nervous system. The autoimmune disease referred to herein may be a Neuromyelitis Optica (NMO) spectrum disorder or multiple sclerosis. The NMO spectrum disorder may be:
1. NMO;
2. a limited form of NMO such as:
   a. Longitudinally extensive tranverse myelitis
   b. Optic neuritis, recurrent or simultaneous bilateral;
3. Asian Optic-Spinal Multiple Sclerosis;
4. Optic Neuritis or Longitudinally Extensive Myelitis Associated With Systemic Autoimmune Disease; or
5. Optic Neuritis or Myelitis Associated With Neuromyelitis Optica-Typical Brain Lesions (from Wingerchuk DM, Lennon VA, Lucchinetti CF, et al. The spectrum of neuromyelitis optica. Lancet Neurol 2007;6(9):805-815.)

The methods according to the present invention relate to the diagnosis, treatment, monitoring and prognosis of an autoimmune disease associated with the formation of demyelinated lesions of the CNS.

Autoimmune disease referred to herein may be characterised by the presence of, or elevated levels of AQP1 autoantibody.

There is also described herein a method of treating a subject comprising withdrawing a body fluid containing AQP1 autoantibodies; removing a substantial portion of the autoantibodies from the body fluid; and returning the body fluid to the subject.

The method of treatment may comprise removing a substantial portion of AQP1 autoantibodies as well as AQP4 autoantibodies.

There is also described herein the use of an AQP1 polypeptide, or a cell or tissue comprising said polypeptide, in an *ex-vivo* method of immunoadsorption of AQP1 autoantibodies.

There is also described herein a solid support for use in a method of immunoadsorption of AQP1 autoantibodies comprising an AQP1 polypeptide, or a cell or tissue comprising said polypeptide, wherein said polypeptide, cell or tissue is bound to the solid support.

The solid support may be, for example, a membrane, a bead, porous glass, silica, a resin or a synthetic matrix.

There is also described herein the use of a solid support described herein in an *ex-vivo* method of immunoadsorption of AQP1 autoantibodies.

There is also described herein a method of identifying compounds capable of alleviating or treating disease characterised by elevated levels of AQP1 autoantibody, the method comprising: contacting a candidate compound in the presence of AQP1 polypeptide and an antibody capable of specifically binding AQP1 wherein a compound that prevents binding of the antibody to AQP1 is a candidate for treating an autoimmune disorder.

There is also described herein an animal model for use in screening for compounds that treat or prevent a disease characterised by elevated levels of AQP1 autoantibodies wherein said animal model expresses or contains AQP1 autoantibodies.

Preferably the animal model is a mouse, rat, guinea pig, rabbit or non-human primate animal model.

The animal may be injected with human anti-AQP1 antibodies. In another embodiment the animal is immunized with the AQP1.

There is also described herein a method of screening for an agent that modulates the binding of an AQP1 antibody to the respective AQP1 polypeptide comprising:
(i) contacting an AQP1 polypeptide with the AQP1 antibody in the presence and absence of said agent; and
(ii) determining whether binding of AQP1 polypeptide to the AQP1 antibody is modulated in the presence of said agent.

According to another aspect of the invention there is provided an AQP1 polypeptide for use in treating a disease characterised by the presence of, or elevated levels of, AQP1 autoantibody wherein the AQP1 polypeptide reduces or eliminates the anti-AQP1 antibodies from the circulation, wherein the disease is an autoimmune disease associated with the formation of demyelinated lesions of the CNS. The AQP1 peptide may be incorporated into a particle such as a nanoparticle or a liposome.

There is also described herein an AQP1 polypeptide for use in immunotherapy for treating a disease characterised by the presence of, or elevated levels of, AQP1 autoantibody wherein the AQP1 polypeptide modulates the immune response against AQP1 in the subject. The AQP1 polypeptide may be incorporated into a particle such as a nanoparticle or a liposome.

There is also described herein a method of treating a subject having a disease characterised by the presence of, or elevated levels of AQP1 autoantibody comprising administration to said subject an agent which binds to the subject's AQP1 and protects it against the pathogenic AQP1 autoantibodies. In one embodiment, the agent may be a non-pathogenic antibody fragment.

There is also described herein the use of AQP1 polypeptide for screening for an agent that modulates the interaction between an AQP1 polypeptide and an AQP1 antibody.

There is also described herein the use of AQP1 antibody for screening for an agent that modulates the interaction between an AQP1 polypeptide and an AQP1 antibody.

There is also described herein a method of diagnosis or prognosis of cancer in a subject wherein the method comprises detecting AQP1 autoantibodies in a sample obtained from said subject.

The method may also involve detecting AQP4 autoantibodies in the same or different sample from said subject.

The cancer may be nephroma, non-Hodgkin lymphoma or mammary cancer.

### Description of Figures

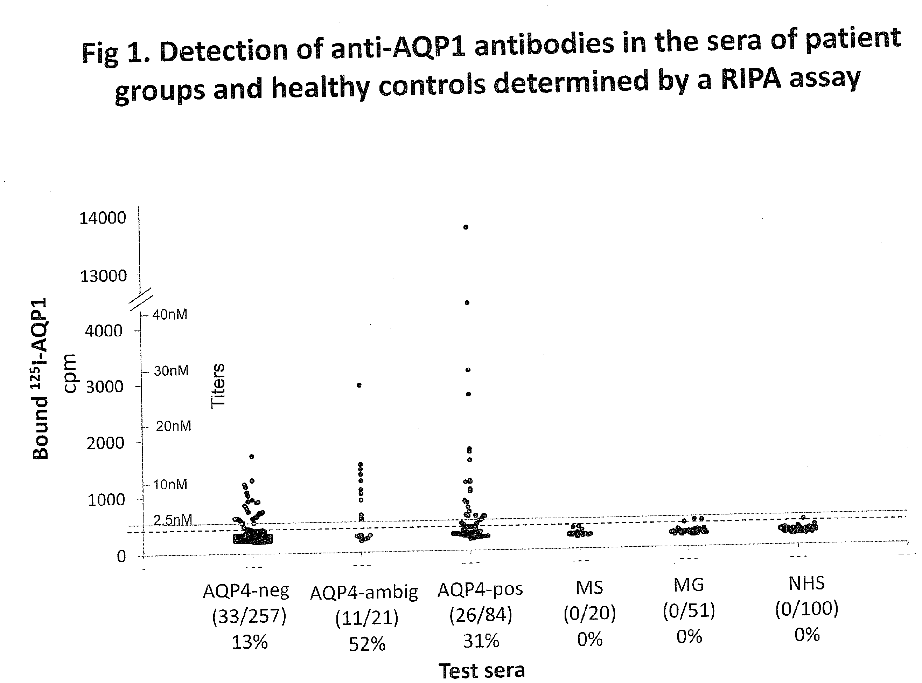

**Figure 1** - Detection of anti-AQP1 antibodies in the sera of patient groups and healthy controls determined by a radioimmunoprecipitation (RIPA) assay
   AQP1 was biotinilated and then incubated with ¹²⁵I-streptavidin to result in indirectly radiolabeled AQP1. Precipitated cpm depicts the level of AQP1 bound to the test serum antibodies and indirectly the level of the autoantibodies in the serum. Numbers in parenthesis at the X-axis denote the numbers anti-AQP1 positive versus total tested sera for each group of patients tested, whereas below is the percentage of the positive. The dashed horizontal line denotes the cut-off values for ambiguous and the solid horizontal line denotes the cut-off values for positivity. The x-axis contains, in addition to the precipitated cpm, also the estimated antibody concentration in nM.

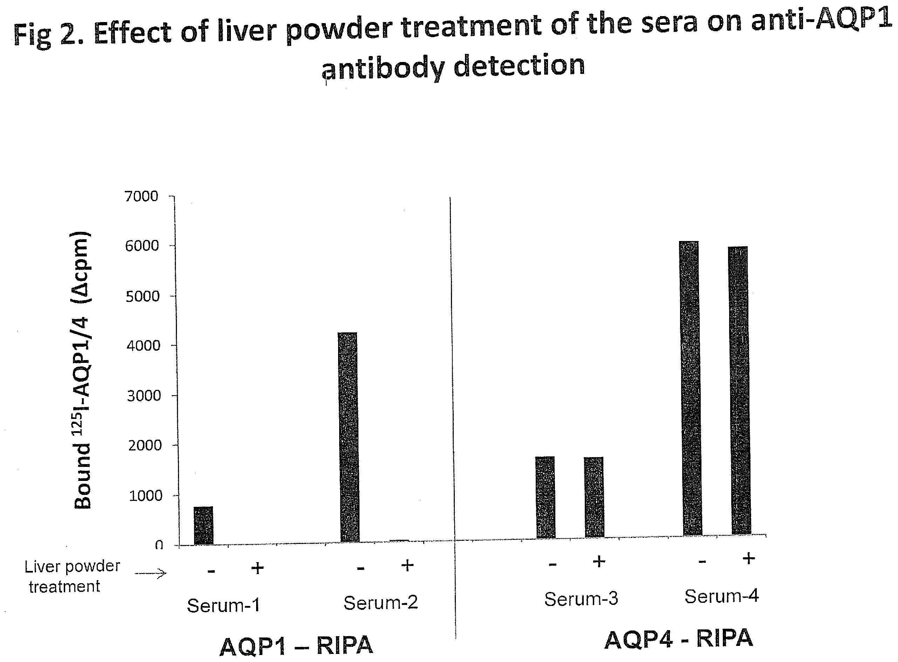

**Figure 2****.** Effect of liver powder treatment of the sera on anti-AQP1 antibody detection
   Two anti-AQP1 positive (sera 1 and 2) and two anti-AQP4 positive sera (sera 3 and 4) were pretreated with guinea pig liver powder; their supernatants were tested in RIA with labeled AQP1 and AQP4, respectively, and compared with those of the untreated sera. -, +: serum without or with pretreatment with guinea pig liver powder.
**Figure 3** - Plotting precipitated cpm values (corresponding to antibody concentrations) in double-positive sera, for anti-AQP1 versus anti-AQP4 antibodies
   Identical RIPAs were performed for the two labeled antigens. Only one serum gave near identical values; 9 sera gave higher values with AQP1 and only 5 sera gave higher values with AQP4.
**Figure 4** - Search for cross-reactivity between the anti-AQP1 and anti-AQP4 antibodies
   AQP1 and AQP4 were immobilized on ELISA wells. Samples of each test serum were incubated with either immobilized aquaporin, AQP1 (right panel) and AQP4 (left panel), in order to immunoadsorb all antibodies bound to AQP1 or AQP4, respectively. The treated 14 sera were tested by RIPA for binding to labeled AQP1 (2^{nd} bar for each serum) and AQP4 (4^{th} bar for each serum), and compared with the binding of the untreated samples of the same sera (1^{st} and 3^{rd} bars for each serum, respectively).

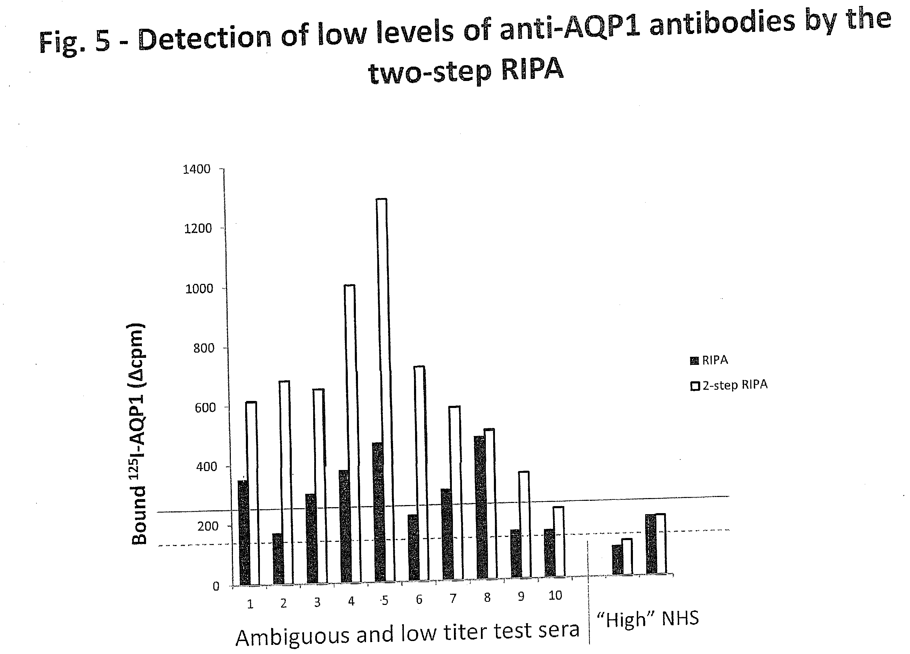

**Figure 5** - Detection of low levels of anti-AQP1 antibodies by the two-step RIPA
   AQP1 was immobilized on each ELISA plate well; 150 µl test serum were added per well and incubated. Then the wells were washed, the bound antibodies were eluted by low pH; the pH of the eluted sample was immediately neutralized and the antibody solution was subjected to regular RIPA. Six low positive sera, 4 ambiguous and 2 high background NHS are shown.
   Δcpm : the bound cpm by the test serum minus the average of the cpm bound by the NHS sera (256 cpm for the direct RIPA and 330 cpm for the two-step RIPA). Dashed and solid horizontal lines denote the cut offs for ambiguous and positive values.

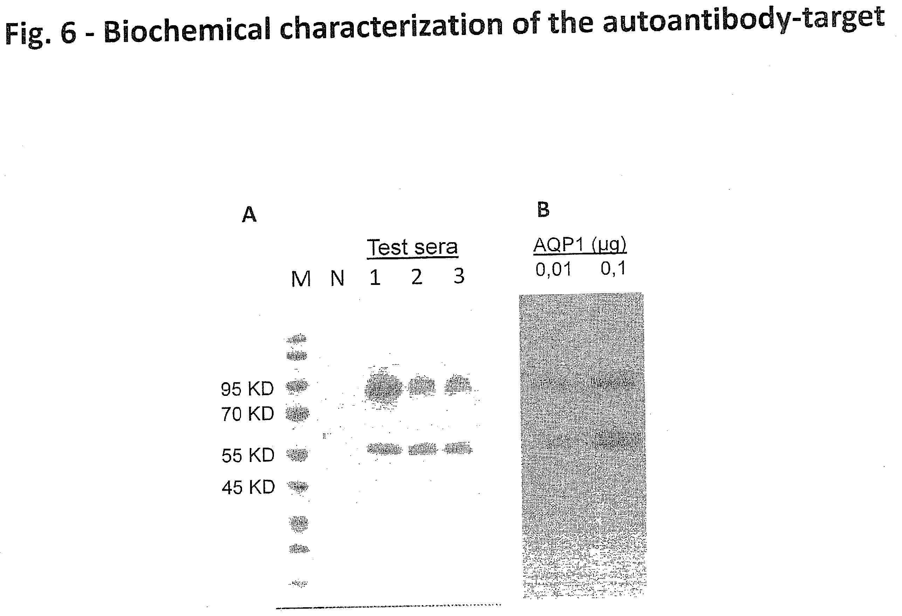

**Figure 6** - Biochemical characterization of the autoantibody-target
   A. The sera (a NHS, labeled as N, and three test AQP1+ sera, labeled as 1,2,3) were incubated with 0.1 µg biotinilated AQP1 followed by precipitation with Sepharose-protein A beads. The bound AQP1 was released, run on PAGE and revealed by western blot using HRP-streptavidin and its substrate. M, MW standards. B. 0,01 and 0,1 µg of biotinilated AQP1 were directly analised by PAGE followed by Western blot in order to semi-quantitate the intensity of AQP1 bands.

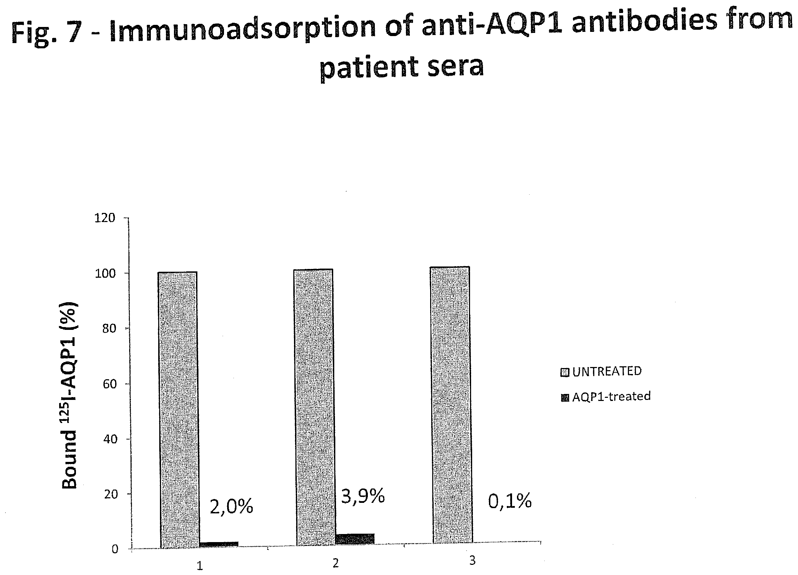

**Figure 7** - Immunoadsorption of anti-AQP1 antibodies from patient sera
   AQP1 was immobilized on ELISA plate well (0,1 µg/well); to the wells was added anti-AQP1 positive serum. After incubation, the treated serum was collected and tested by RIPA with indirectly ¹²⁵I-labeled AQP1. In parallel, an equal quantity of the untreated serum was tested by RIPA in order to determine the percentage of the serum anti-AQP1 antibodies which were eliminated from the treated serum.

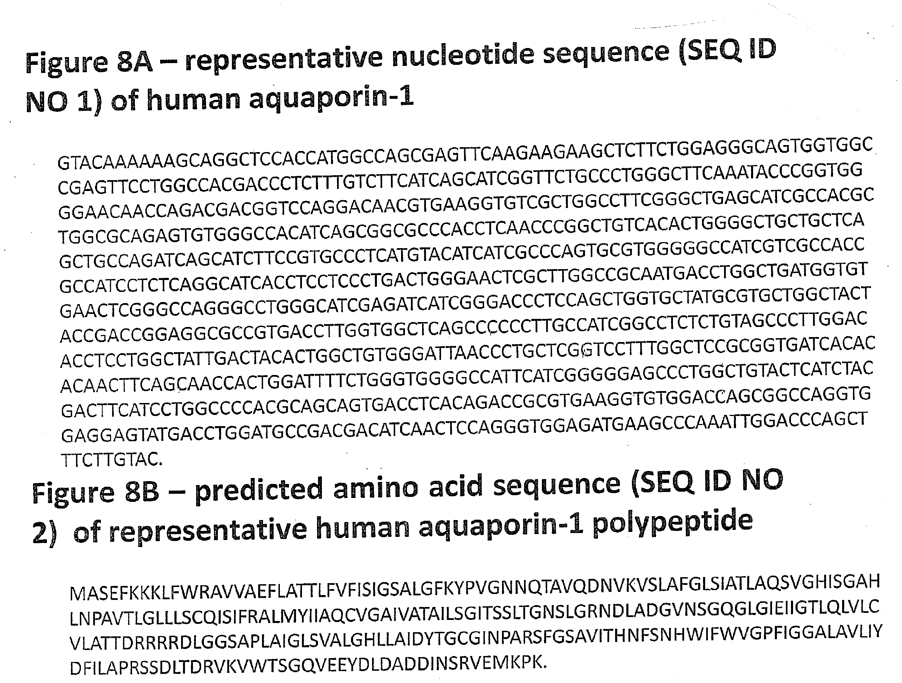

**Figure 8** - Representative sequence of human aquaporin-1

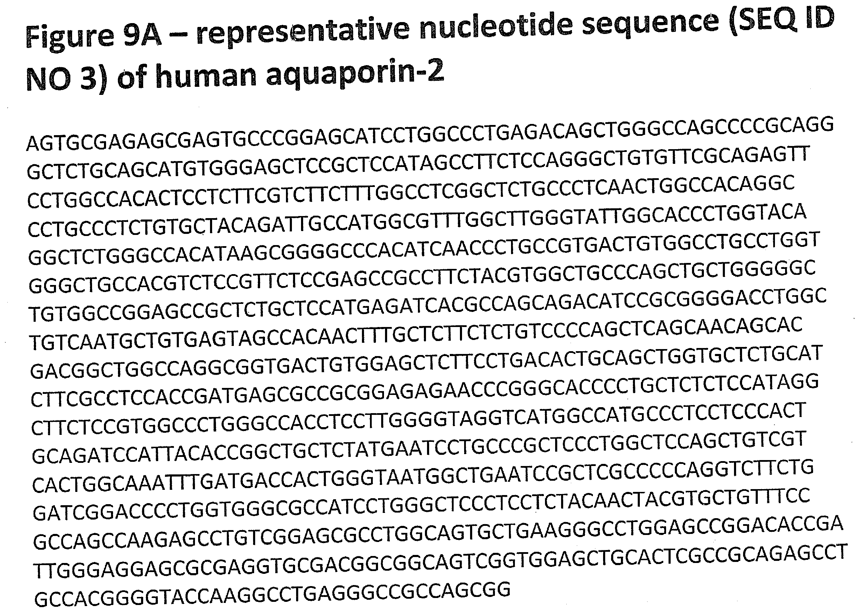

**Figure 9A** - Representative nucleotide sequence of human aquaporin-2. This sequence is also shown in GenBank Accession number: Z29491.

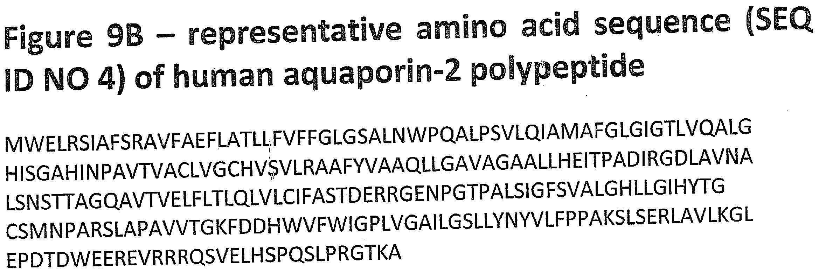

**Figure 9B** - Representative amino acid sequence of human aquaporin-2 polypeptide. This sequence is also shown in GenBank Accession number: AAD38692.1.

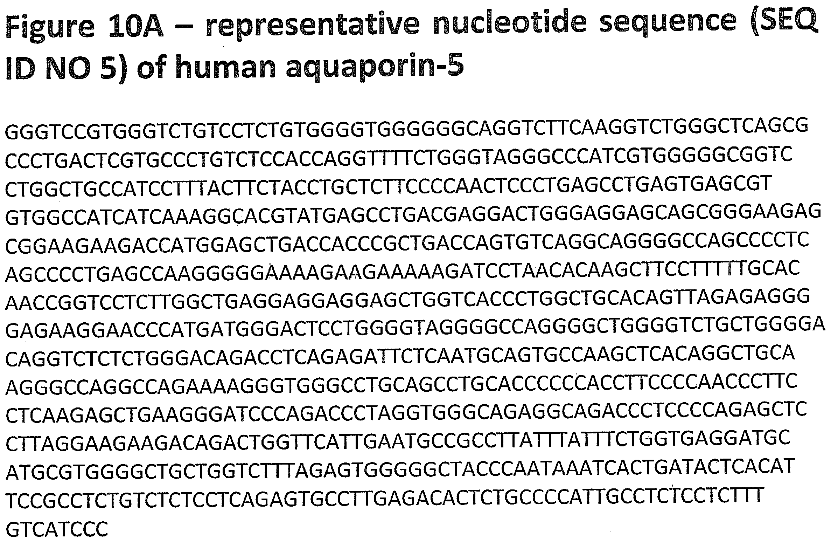

**Figure 10A** - Representative nucleotide sequence of human aquaporin-5. This sequence is also shown in GenBank Accession number: U46569.

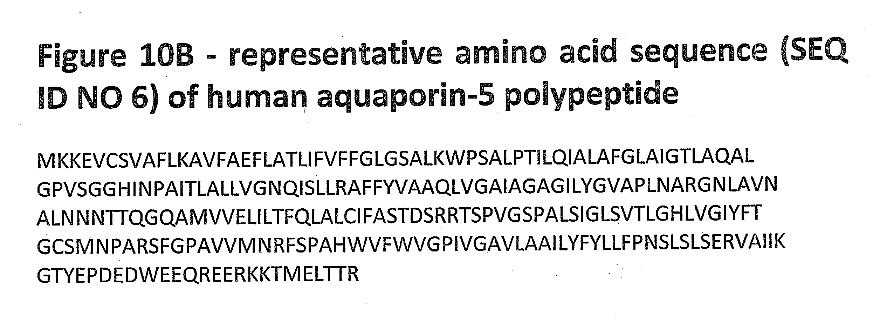

**Figure 10B** - Representative amino acid sequence of human aquaporin-5 polypeptide. This sequence is also shown in GenBank Accession number: AAH32946.1.

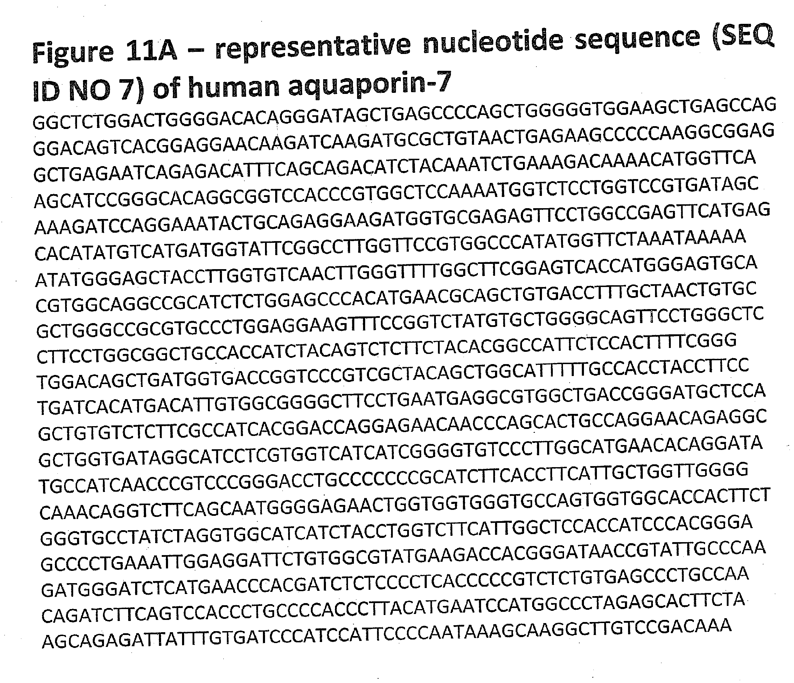

**Figure 11A** - Representative nucleotide sequence of human aquaporin-7. This sequence is also shown in GenBank Accession number: AB006190.

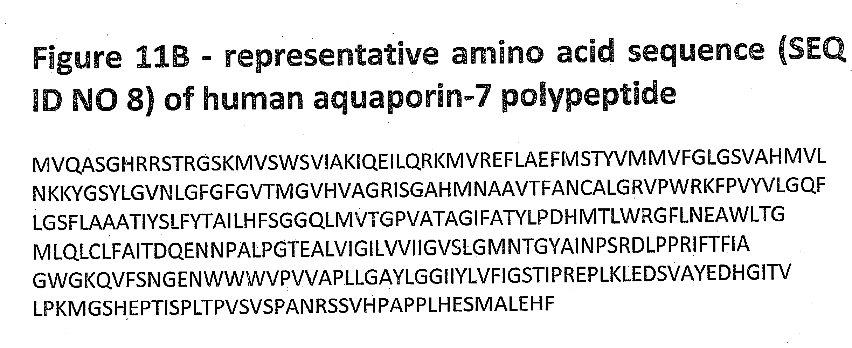

**Figure 11B** - Representative amino acid sequence of human aquaporin-7 polypeptide. This sequence is also shown in GenBank Accession number: CAI13309.1.

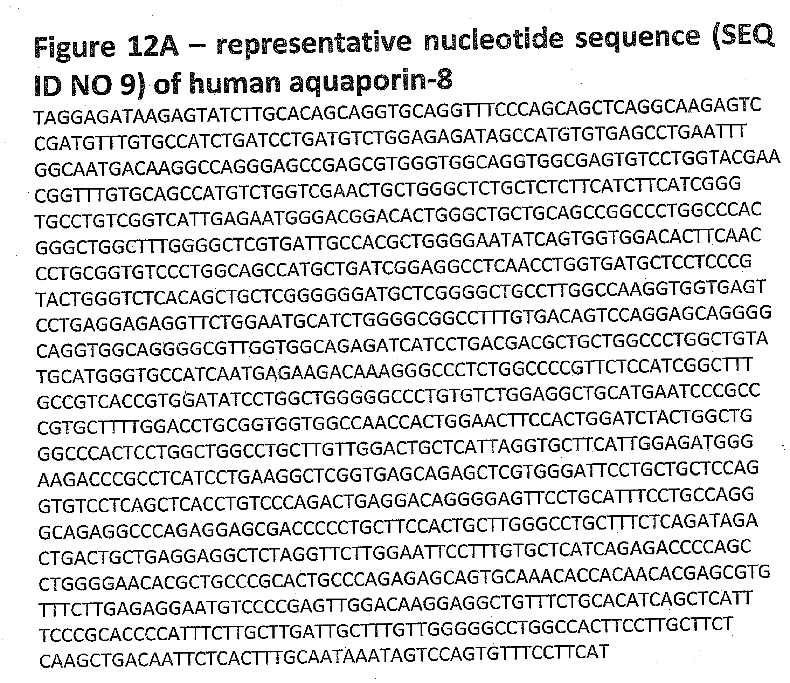

**Figure 12A** - Representative nucleotide sequence of human aquaporin-8. This sequence is also shown in GenBank Accession number: AB013456. T

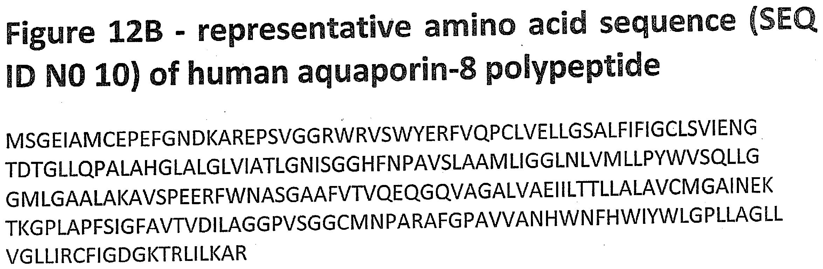

**Figure 12B** - Representative amino acid sequence of human aquaporin-8 polypeptide. This sequence is also shown in GenBank Accession number: AAF19050.1.

### Detailed description

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Col d Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; and E. M. Shevach and W. Strober, 1992 and periodic supplements, Current Protocols in Immunology, John Wiley & Sons, New York, NY.

Aquaporin proteins are made up of six transmembrane α-helices arranged in a right-handed bundle, with the amino and the carboxyl termini located on the cytoplasmic surface of the membrane (Gonen T, Walz T (2006) "The structure of aquaporins". Q. Rev. Biophys. 39 (4): 361-96; Fu D, Lu M (2007) "The structural basis of water permeation and proton exclusion in aquaporins", Mol. Membr. Biol. 24 (5-6): 366-74).

The amino and carboxyl halves of the sequence show similarity to each other, in what appears to be a tandem repeat. There are also five interhelical loop regions (A - E) that form the extracellular and cytoplasmic vestibules. Loops B and E are hydrophobic loops that contain the highly, although not completely conserved, asparagine-proline-alanine (NPA) motif, which overlap the middle of the lipid bilayer of the membrane forming a 3-D 'hourglass' structure where the water flows through. This overlap forms one of the two well-known channel constriction sites in the peptide, the NPA motif and a second and usually narrower constriction known as 'selectivity filter' or ar/R selectivity filter.

Aquaporins form tetramers in the cell membrane, with each monomer acting as a water channel (Gonen T, Walz T (2006) Q. Rev. Biophys. 39 (4)). The different aquaporins contain differences in their peptide sequence, which allows for the size of the pore in the protein to differ between aquaporins. The resultant size of the pore directly affects what molecules are able to pass through the pore, with small pore sizes only allowing small molecules like water to pass through the pore.

Examples of a nucleotide sequence encoding a human aquaporin-4 polypeptide are shown in GenBank Accession Nos. U63622 and U63623. The predicted amino acid sequences of representative human aquaporin-4 polypeptides are shown in GenBank Accession Nos. AAG17964, AAB26957, AAB26958, and 139178. Nucleic acid and amino acid sequences encoding aquaporin-4 from other organisms can be found by searching the GenBank database (www.ncbi.nlm.nih.gov) using "aquaporin-4" as the search word.

Example of a nucleotide sequence encoding a human aquaporin-1 polypeptide Is shown in GenBank Accession Nos. AAH22486. The predicted amino acid sequence of representative human aquaporin-1 polypeptide is shown in ProteinBank Accession Nos. AAX24129.1. Nucleic acid and amino acid sequences encoding aquaporin-1 from other organisms can be found by searching the GenBank database (www.ncbi.nlm.nih.gov) using aquaporin-1 as the search word.

Examples of a nucleotide sequence encoding human aquaporins -2, - 5 -7-8 polypeptide are shown in GenBank Accession No. Z29491, U46569, AB006190 and AB013456 respectively. The representative amino acid sequence of human aquaporins -2, - 5 -7, -8 polypeptide is shown in GenBank Accession No. AAD38692.1, AAH32946.1, CAI13309.1 and AAF19050.1 respectively. Nucleic acid and amino acid sequences encoding aquaporins -2, - 5 -7, -8 from other organisms can be found by searching the GenBank database (www.ncbi.nlm.nih.gov) using "aquaporins -2, - 5 -7, -8" as the search words.

A "protein" or "polypeptide" is understood within the scope of the invention to include single-chain polypeptide molecules, as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulfide bonds. Portions of the polypeptide may be referred to as a "subunit" or a "domain" or "fragment" as applicable. The term polypeptide encompasses polypeptides that have been modified. The term polypeptide also encompasses variants, derivatives, analogues and homologues.

The AQP1 polypeptide referred to herein encompasses variants, derivatives, analogues and homologues of AQP1 for which AQP1 autoantibodies have specific affinity. Similarly, a fragment of an AQP1 polypeptide referred to herein encompasses fragments for which AQP1 autoantibodies have specific affinity.

Similarly, the aquaporin-2 (AQP2), aquaporin-5 (AQP5), aquaporin-7 (AQP7) and aquaporin-8 (AQP8) polypeptides referred to herein encompass variants, derivatives, analogues and homologues of AQP2, AQP5, AQP7 and AQP8 for which respective AQP2, AQP5, AQP7 and AQP8 autoantibodies have specific affinity. Similarly, fragments of AQP2, AQP5, AQP7 and AQP8 polypeptides referred to herein encompass fragments for which respective AQP2, AQP5, AQP7 and AQP8 autoantibodies have specific affinity.

The AQP1 autoantibodies may bind (e.g. via the antigen-specific binding site(s) or paratopes of the antibody, which are present within the variable regions) to an antigenic epitope present within the AQP1 polypeptide. Similarly, the AQP2, AQP5, AQP7 and AQP8 autoantibodies may bind (e.g. via the antigen-specific binding site(s) or paratopes of the antibody, which are present within the variable regions) to an antigenic epitope present within the respective AQP2, AQP5, AQP7 and AQP8 polypeptides. Typically the antibody may bind to the respective AQP1, AQP2, AQP5, AQP7 or AQP8 polypeptide or fragment with high affinity, e.g. with a dissociation constant (K_{d}) of less than 1 µM, preferably less than 10nM, preferably less than 1 nM. Preferably the AQP1 antibody specifically binds to AQP1 and does not significantly bind unrelated antigens. Preferably the AQP2 antibody specifically binds to AQP2 and does not significantly bind unrelated antigens. Preferably the AQP5 antibody specifically binds to AQP5 and does not significantly bind unrelated antigens. Preferably the AQP7 antibody specifically binds to AQP7 and does not significantly bind unrelated antigens. Preferably the AQP8 antibody specifically binds to AQP8 and does not significantly bind unrelated antigens.

AQP1, AQP2, AQP5, AQP7 and AQP8 polypeptides or fragments thereof include one or more epitopic sites. Computer algorithms are available for predicting binding epitopes, e.g., MHC-I and MHC-II binding epitopes. See, for example, http://bimas.dcrt.nih.gov:80/molbio/hla_bind/ (Parker et al., J. Immunol., 152:163 (1994); Southwood et al., J. Immunol., 160:3363 (1998)).

B-cell epitopes of a protein are mainly localised at its surface. To predict B-cell epitopes, two well-known methods may be used: 2D-structure prediction and antigenic index prediction. The 2D-structure prediction can be made using the psipred program (from David Jones, Brunel Bio-informatics Group, Dept. Biological Sciences, Brunel University, Uxbridge UB8 3PH, UK). The antigenic index can be calculated on the basis of the method described by Jameson and Wolf (CABIOS 4: 181-186 [1988]). In addition, as explained on page 73, lines 2 to 5, the prediction of useful T-helper cell epitopes can be performed using the T-epitope method described by Sturniolo et al (Nature Biotech. 17: 555-561 [1999]). Hirschberg et al (APMIS Volume 99, Issue 1-6, pages 515-520, January 1991) demonstrates the use of these techniques to generate peptide fragments of the *Mycoplasma pneumoniae* P1 protein which were recognised by antibodies in the serum of patients.

The epitopic site allows immunologic detection of AQP1, AQP2, AQP5, AQP7 and AQP8 autoantibodies in sera with reasonable assurance. Usually, it is desirable that the epitopic site be antigenically distinct from other closely related antigens (e.g., other members of a family of polypeptides). A representative antigenic fragment can include, for example, the extracellular domain of a membrane-bound protein.

A variant or derivative sequence can be obtained by addition, deletion, substitution, modification, replacement and/or variation of at least one residue present in the naturally-occurring protein.

The term "analogue" as used herein, in relation to polypeptides includes any mimetic, that is, a chemical compound that possesses at least one of the endogenous functions of the polypeptides which it mimics.

Typically, amino acid substitutions may be made, for example from 1, 2 or 3 to 10 or 20 substitutions provided that the modified sequence retains the required activity or ability. Amino acid substitutions may include the use of non-naturally occurring analogues.

Polypeptides for use in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the transport or modulation function is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other.

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

The AQP1, AQP2, AQP5, AQP7 and/or AQP8 polypeptides and fragments may be labeled directly or indirectly with a second substance that provides for detectable signal. Examples of labels include, but are not limited to radioisotopes, enzymes, substrates, cofactors, inhibitors, fluorescers, chemiluminescers, magnetic particles, and the like.

The presence of AQP1 autoantibodies can be used to diagnose diseases associated with the presence or elevated expression of said antibodies, such as diseases characterised by demyelinated lesions, in particular demyelinated lesions of the CNS or of the peripheral nervous system, at an early stage of the disease before all clinical criteria are fulfilled, thus justifying early initiation of appropriate immunosuppressant therapy. The presence of AQP1 autoantibodies may also be used to distinguish the autoimmune disease from other autoimmune diseases such as, but not limited to classic MS and MG.

Detecting AQP1 autoantibodies also can provide a quantifiable biomarker for monitoring disease progression and response to therapy. In addition, the identification of an antibody associated with AQP1 provides a valuable tool for developing animal models (e.g., by passive transfer of NMO-specific antibodies or active immunization with AQP1 antigenic polypeptides or DNA vaccines encoding such antigenic polypeptides) to investigate the pathogenesis of diseases characterised by AQP1 autoantibody expression and to test potential new therapies.

The present disclosure provides for methods of detecting AQP1 specific autoantibodies in an individual. Individuals for whom the methods of the invention might be used typically may present abnormal neurological symptoms including, but not limited to, tingling, numbness, weakness, limb spasms, limb paralysis, sensory loss, radicular pain, diplopia and nystagmus, vision impairment, loss of bladder and/or bowel control, or other neurological symptoms of unknown origin.

Further provided by the disclosure are kits containing an AQP1 polypeptide or fragment thereof. The kit may further include a second substance that provides for detectable signal. A kit typically also includes directions for using the AQP1 polypeptide for detecting the AQP1-specific autoantibodies in a biological sample.

The method describes an association between the presence of abnormal levels or pattern of expression of the AQP1, the subsequently produced AQP1-specific autoantibody or autoantibodies, and the resulting disorder in a subject.

Antibodies may be produced by standard techniques. Polyclonal, monoclonal, chimeric, single chain, Fab fragments, fragments produced by a Fab expression library, as well as mimetics thereof may be used in the present invention. Such fragments include fragments of whole antibodies which retain their binding activity for a target substance, Fv, F(ab') and F(ab')₂ fragments, as well as single chain antibodies (scFv), fusion proteins and other synthetic proteins which comprise the antigen-binding site of the antibody. Furthermore, the antibodies and fragments thereof may be humanised antibodies.

If polyclonal antibodies are desired, a selected mammal (e.g., mouse, rabbit, goat, horse, etc.) is immunised with the relevant antigen. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG (*Bacilli Calmette-Guerin*) and *Corynebacterium parvum* are potentially useful human adjuvants which may be employed if purified the substance polypeptide is administered to immunologically compromised individuals for the purpose of stimulating systemic defence.

Serum from the immunised animal is collected and treated according to known procedures. The polyclonal antibodies can be purified by immunoaffinity chromatography. Techniques for producing and processing polyclonal antisera are known in the art.

Monoclonal antibodies can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against orbit epitopes can be screened for various properties; i.e., for isotype and epitope affinity.

Monoclonal antibodies may be prepared using any technique which provides for the production of antibody molecules by continuous cell lines in culture. These include, but are not limited to, the hybridoma technique originally described by Koehler and Milstein (Koehler and Milstein, 1975), the human B-cell hybridoma technique (Kosbor et al., 1983; Cote et al., 1983) and the EBV-hybridoma technique (Cole et al., 1985). In addition, techniques developed for the production of "chimeric antibodies", the splicing of mouse antibody genes to human antibody genes to obtain a molecule with appropriate antigen specificity and biological activity can be used (Morrison et al., 1984; Neuberger et al., 1984; Takeda et al., 1985). Alternatively, techniques described for the production of single chain antibodies (US Patent No. 4,946,779) can be adapted to produce the substance specific single chain antibodies.

Within certain embodiments, the use of antigen-binding fragments of antibodies may be preferred. Such fragments include Fab fragments, which may be prepared using standard techniques, for example digestion by papain to yield Fab and Fc fragments which may be separated by affinity chromatography.

A "sample" is understood within the scope of the invention to refer to a sample which is derived from a subject. The biological sample may be obtained directly from the subject or may be derived from cultured cells obtained from said subject. Preferred samples are those derived from blood which may comprise plasma, serum or fractions such as an immunoglobulin fraction. The biological sample may have been subjected to a treatment such as dilution in a carrier.

Assays used in the diagnosis or prognosis methods described herein include, but are not limited to, an immunoassay, a ligand-binding assay, a surface plasmon resonance assay or a microchip-based assay or any other assay commonly known in the art. Details regarding such assays can be found in known literature and periodic supplements, such as Current Protocols in Immunology, Copyright © 2010 by John Wiley and Sons, Inc, Last Updated: February 08, 2010, Print ISSN: 1934-3671 (see e.g. chapters 2, 6 and 10).

An "immunoassay" is understood within the scope of the invention to include an enzyme-linked immunosorbent assay (ELISA), radioimmunoassay (RIA), immunoprecipitation assay, immunofluorescence assay (IFA), indirect immunofluorescence assay (IIF), enzyme linked assay (EIA), luminescence immunoassay (LIA), Western Blot assay (WB) and cell based assay (CBA) or any other immunoassay commonly known in the art.

Further described herein are methods of treating a subject having a disease characterised by the presence of, or elevated expression of, AQP1 antibodies. Methods of treating a subject include, but are not limited to, apheresis and T cell receptor-based immunotherapy.

Methods and extracorporeal systems for apheresis (i.e., the process of withdrawing blood from an individual, removing components from the blood, and returning the blood, or blood depleted of one or more components, to the individual) are known in the art (see, for example, U.S. Pat. Nos. 4,708,713; 5,258,503; 5,386,734; and 6,409,696). The present invention provides a method of removing AQP1-specific autoantibodies from a body fluid of an individual. The method involves withdrawing a body fluid from a subject; removing a substantial portion of AQP1-specific autoantibodies from the fluid; and returning the fluid to the subject. Antibodies removed can be of any class, e.g., IgG (such as IgG1, IgG2, IgG3, IgG4), IgM, IgD, IgA, or IgE antibodies.

As used herein, a "substantial portion" means removing at least 20%, 30%, 40%, 50%, 60%, 70%, 80%, 90%, 95%, 96%, 97%, 98%, 99%, or even 100% of the AQP1, AQP2, AQP5, AQP7 or AQP8 -specific autoantibodies that were present in the body fluid prior to removal. The body fluid can be blood plasma or any other body fluid, e.g., lymph or cerebrospinal fluid.

Removal of AQP1, AQP2, AQP5, AQP7 and/or AQP8 -specific autoantibodies may be performed by contacting a body fluid with an AQP1, AQP2, AQP5, AQP7 and/or AQP8 polypeptide or fragment thereof. The AQP1, AQP2, AQP5, AQP7 and/or AQP8 polypeptide or fragment can be bound to a solid support. Such solid supports can be, without limitation, membranes, fibres, spherical beads, or granules and can be made with a water-insoluble, preferably porous, biocompatible material, e.g., organic polymers such as agarose, dextran, and polyacrylamide, or inorganic porous materials such as porous glass or porous silica gel. Such materials are suitable or can be adapted (e.g., derivatized with appropriate chemical groups) for attachment of AQP1, AQP2, AQP5, AQP7 and/or AQP8 polypeptide or fragment thereof.

An effective amount of an AQP1 polypeptide may be administered to a subject to modulate the subject's AQP1-mediated immune response. Thus the AQP1 polypeptide may be used in immunotherapy to treat a disorder characterised by the presence of AQP1 autoantibodies.

Thus, there is described herein a pharmaceutical composition comprising an AQP1 polypeptide or fragment.

A pharmaceutical composition described herein may optionally comprise a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system).

Where appropriate, the pharmaceutical compositions can be administered by any one or more of: inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

Methods to deliver a composition to the brain are known in the art. For example, a composition of the invention can be modified by attaching a ligand (e.g., an antibody or antibody fragment) that recognizes a brain-specific or neuron-specific receptor. In addition, methods of enhancing transport of molecules across the blood-brain barrier are known, and take advantage of passive diffusion (e.g., using electromagnetic fields, nitric oxide donors or sodium caprate) or receptor-mediated endocytosis (e.g., attachment of the virus particle to, for example, an anti-transferrin antibody or to putrescine). Brain-specific or neuron-specific promoter and/or transcriptional regulatory elements may also be used (see, for example, U.S. Pat. No. 5,976,872 or 6,066,726).

A "subject" refers to either a human or non-human animal. Examples of non-human animals include vertebrates, e.g., mammals, such as non-human primates (particularly higher primates), dogs, rodents (e.g., mice, rats, or guinea pigs), pigs and cats, etc. In a preferred embodiment, the subject is a human.

Further preferred features and embodiments of the present invention will now be described by way of the following non-limiting examples.

### Example 1 - Methods

### Patient sera

Sera of 362 patients for whom test for anti-AQP4 antibodies was requested by their doctors, were used after informed consent; The selection of these sera was as follows: 297 sera were from almost all patients who requested in our diagnosis unit testing for NMO antibodies, independently of their anti-AQP4 outcome (35 of these sera were anti-AQP4 positive and 5 ambiguous). The remaining 65 sera were selected as either anti-AQP4 positive (49 sera) or anti-AQP4 ambiguous (16 sera). 110 of the 362 sera were from patients evaluated clinically at the Aiginiteion University Hospital. In addition to the above 362 sera, 20 patients with classical MS, 51 MG patients (positive for anti-acetylcholine receptor antibodies), and 100 sera from healthy controls were also tested for the presence of anti-AQP1 antibodies. Retrospective analysis, of MRI, clinical, cytologic and statistical data, from patients positive for anti-AQP1 antibodies with available data followed.

### AQP1, AQP2, AQP5, AQP4, AQP7 and AQP8

Human recombinant AQP4 with 6-his tag was expressed in Pichia pastoris expression system and purified by Ni-column chromatography and gel filtration. Human recombinant AQP1 with glutaphion-S-transferase (GST) tag was purchased from Novus Biologicals; it was expressed in wheat germ cell-free protein expression system and purified by affinity purification with immobilized glutathione. Human recombinant AQP2, AQP7, and AQP8 proteins with a glutathione-S-transferase (GST) tag expressed in the wheat germ cell-free protein expression system and purified by affinity chromatography were purchased from Novus Biologicals, Cambridge, UK. AQP5 cDNA clone was purchased from OriGene Technologies Inc, Rockville, MD, and was transiently expressed in HEK293 cells.

### Radioimmunoprecipitation assay (RIPA)

RIPA usually involves the incubation of the test serum with radiolabeled antigen, followed by the addition of an anti-Ig serum to make an Ig/anti-Ig precipitate; if the test serum has antibodies against the labeled antigen, the latter will be coprecipitated with the Ig complex resulting in radioactive precipitate. In our RIPA, recombinant human AQP1 or AQP4 was ¹²⁵I-labeled indirectly as follows: 0.25 nmole AQP were incubated with 5 nmole biotin. The biotinilated AQP was indirectly labeled by incubation with ¹²⁵I-streptavidin at a ratio: 0.1 µg biotinilated-AQP with 50,000 cpm ¹²⁶I-streptavidin, for 1 hr on ice. For the RIPA, 5 µl (or less) of test serum were incubated for 2h at 25 °C with 50 µl phosphate buffered saline (PBS)-0.5% Triton X-100 buffer, with 0.1 µg of the indirectly labeled AQP. After 2h incubation at room temperature, 50 µl of goat anti-human immunoglobulin antiserum was added and after incubation for 2 h at 25 °C the formed pellet was washed twice with 1 ml of assay buffer (0.5% Triton X-100 in PBS). The radioactivity in the washed pellet was counted in a γ-counter.

The average of the cpm values for the healthy human control sera (256 cpm), plus 3SD (3x35 cpm), total 361 cpm, multiplied by 1.4 (total 361X1.4= 505 cpm) was considered the cut-off for positive sera whereas multiplied by 1.1 (361X1.1= 397 cpm) was considered the cut-off for "ambiguous" sera. Each 100 cpm above the background corresponds to 1 nM antibody when using 5 µl serum in the assay, therefore the ambiguous and positive cutoffs correspond to 1.4 nM (397 cpm = 256 cpm average background + 141 extra cpm; 141/100 = ∼1.4 nM) and 2.5 nM (505 cpm = 256 cpm average background + 249 extra cpm; 249/100 = ∼2.5 nM) antibody concentrations.

Ten sera positive in this assay were also tested for binding to the same amount of ¹²⁵I-streptavidin in the absence of biotinilated aquaporin. They precipitated background cpm values, similar to those of the NHS controls, confirming that the antibodies indeed bind to the AQP1-GST rather than to the labeled streptavidin. To exclude the possibility that some antibodies bound to the GST moiety of the AQP1-GST fusion protein, 9 anti-AQP1 and 4 anti-AQP4 sera were preincubated for 3 hr with excess GST immobilized on Sepharose-glutathione beads (10 µl serum with 1.6 µg immobilized GST); then 5 µl of the treated sera were tested in RIPA with labeled AQP1 or AQP4. No decrease in the bound cpm was observed confirming that the patients' antibodies indeed bind to the AQP1 part of the fusion protein.

Finally, in order to verify the reason that AQP1-positive sera pretreated with liver powder cannot give positive result in IIF, two anti-AQP1 and two anti-AQP4 serum samples (5 µl each diluted in 100 µl buffer) were pretreated with guinea pig liver powder (20 mg each) for 1 h. After centrifugation the supernatants were tested with labeled AQP1 and AQP4 respectively and the precipitated cpms were compared with those of the untreated sera (similarly diluted).

### Two-step RIPA

In selective cases, a more sensitive radioimmunoassay, based on a general very sensitive immunoassay method we developed, as described in PCT/IB2011/001014, involving two steps (two-step RIPA) was also used:
Step 1: ELISA plates were plated with 50 µl carbonate buffer pH 9.6 containing 0.1 µg AQP1 or AQP4 per well, incubated for 2 hr at 25 °C , followed by saturation with 1% PBS-bovine serum albumin (PBS-BSA) for 1 h at 25 °C. Then, 0.15 ml of test serum was added to each well and the sample incubated overnight at 4 °C with constant rotation. The wells were sequentially washed 3 times with PBS and once with 0.1 M glycine-HCl, pH 6. Then 50 µl of 0.1 M glycine-HCI buffer, pH 2.5, was added to the washed wells and shaked for 1 min. The liquid, containing the released autoantibodies was then transferred to test tubes containing 10 µl of 0.375 M Tris buffer, pH 8.8, in order to immediately neutralize the pH.
Step 2: To the above mixture, 20 µl of buffer containing 0.1 µg biotinilated AQP and 50.000 cpm ¹²⁵I-labeled streptavidin) and 2 µl of carrier NHS were added. After 6 h incubation at room temperature, 50 µl of goat anti-human immunoglobulin antiserum was added. After incubation for 2 h, the formed pellet was washed twice with 1 ml of PBS-0.5% Triton X-100 and the radioactivity of the washed pellet counted in a γ-counter.

### Characterization of the autoantigen by immunoprecipitation and Western blot assay

Anti-AQP1 and anti-AQP4 positive sera and NHS were incubated with 0.1 µg preparation of unlabeled biotinilated AQP1 or AQP4 respectively. Then, the mixtures were incubated with 10 µl Sepharose beads with immobilized protein A. The beads were subsequently washed, treated with sample buffer for PAGE containing 2% SDS, 50 mM Tris-HCI pH 6.8, 10% glycerol, 0.1% bromophenol blue and 2% β-mercaptethanol to release the bound antibodies with any bound biotinilated AQP and subjected to 4-12% gradient polyacrylamide gel electrophoresis (PAGE) and subsequently blotted to nitrocellulose membrane. The detection was accomplished by incubating the membranes with 1/500 dilution of HRP-streptavidin for 16 h at 4 °C followed by saturation with 3% PBS-BSA for 1 h at 25 °C.

In parallel, 0.01 and 1 µg of biotinilated AQP1 were directly run on PAGE, blotted and detected as above with HRP-streptavidin in order to semi-quantitate the protein.

### Indirect immunofluorescence

Immunofluorescence microscopy on frozen nonpathological mouse whole brain sagital sections on slides is performed. Specifically, sections are incubated with 10% formalin in PBS for 4 min for fixation, followed by three washes in PBS. Next, blocking is performed, using 10% normal horse serum in PBS for 1 hr. Then, sections are incubated with patient and control sera (1:60) for 1 hr at room temperature. After washing the section three times with PBS, the secondary antibody, Alexa-488 goat-anti-human IgG (1/200 dilution) is applied for 1 hr at room temperature, washed with PBS three times, and viewed on a confocal microscope. Seropositivity is reported when IgG bound selectively to AQP1-rich tissues. Contrary to previously standardized method for detecting anti-NMO IgG (Lennon et al. (2004) Lancet 364(9451): 2106-2112), serum is not preabsorbed with liver powder in order to avoid elimination of the anti-AQP1 antibodies. To overcome the background problem, anti-AQP1 antibodies are preferably, but not necessarily, partially purified by affinity chromatography on ELISA plates with plated AQP1.

### Retrospective analysis of available MRI and clinical data

For 20 anti-AQP1 positive sera (5 anti-AQP4 positive and 15 anti-AQP4 negative) and 9 anti-AQP1 ambiguous (the 8 of which were anti-AQP4-negative) detailed clinical data of the corresponding patients could be obtained. These included: MRI (brain, spinal cord and optic nerves), clinical (CNS, periphery, etc) and cytological (plasma albumin and CSF) study.

### Example 2 - Antibodies specific for AQP1 are present in patients suspected for NMO or NMO-like symptoms

We examined sera from 362 patients, suspected for NMO, with RIPA for anti-AQP1 antibodies. Previously these sera were examined for anti-AQP4 antibodies with the more sensitive two-step RIPA and had identified 84 of them as anti-AQP4 positive. We found in total 70 sera positive for AQP1 antibodies (Fig. 1). We found 44 AQP1-positive sera out of the 278 AQP4-negative or ambiguous (AQP1+/AQP4-; 16%) and 26 AQP1-positive sera out of the 84 AQP4-positive (AQP1+/AQP4+; 31%). The M/F ratios were 1:2.4 (13/31) for the AQP1+/AQP4- patients, 1:4.2 (5/21) for the AQP1+/AQP4+ patients, and 1:4.3 (11/47) for the AQP1-/AQP4+ patients. I.e. males are considerably more frequent in the exclusively AQP1+ group of patients than in the AQP4+ group (with or without anti-AQP1 antibodies), although females remain more frequent in both disease groups.

Fig 1 also shows that 20 sera from patients with classical MS, 51 sera of MG patients, positive for anti-AChR antibodies, and 100 sera from healthy controls were all negative for anti-AQP1 antibodies (with 0, 3 and 1 sera characterized as ambiguous, respectively). These data suggest that anti-AQP1 antibodies are a specific biomarker for a population of patients with demyelinated lesions.

Two anti-AQP1 positive and two anti-AQP4 positive sera were pretreated with guinea pig liver powder; their supernatants were tested with labeled AQP1 and AQP4, respectively, and compared with those of the untreated sera. It was found that the liver powder practically completely (by 99-100%) adsorbed the anti-AQP1 antibodies whereas it did not adsorb at all the anti-AQP4 antibodies (Fig 2). This finding is based on the known fact that liver powder contains AQP1, but not AQP4, and shows clearly why AQP1-positive sera pretreated with liver powder cannot easily give positive result in IIF. Thus, autoantibodies to AQP1 usually might not be detected with the usual IIF assay on mouse tissues used for NMO-IgG antibodies because this assay, in order to reduce background, usually involves treatment of the test serum with guinea pig liver powder, which incidentally contains AQP1 (Talbot et al. (2003) Cells Tissues Organs 174(3): 117-128) and therefore any autoantibodies to AQP1 in the test serum would be eliminated and not detected.

### Example 3 - There is no correlation between anti-AQP1 and anti-AQP4 concentrations in double positive (anti-AQP1+/anti-AQP4+) sera

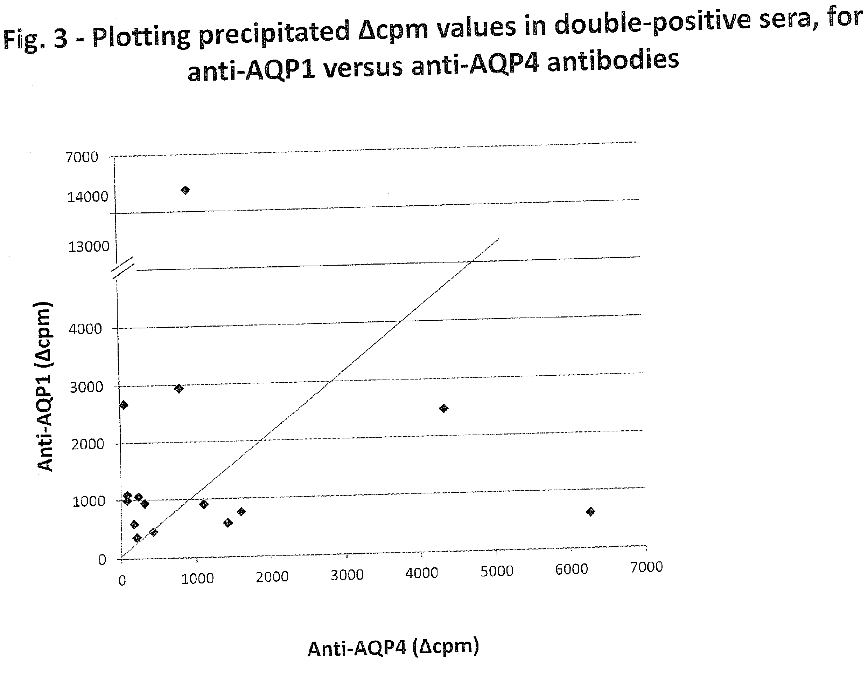

We then tested whether, in the double positive sera, there is any correlation of antibody concentrations between the two groups of autoantibodies, which would be an indication of linked immune response. Fig. 3 plots the anti-AQP1 versus the anti-AQP4 antibody levels as tested by regular RIPAs. It is clear that there is no correlation between the levels of the two antibody groups in the double positive sera. Furthermore, in most cases (9 versus 5) the anti-AQP1 levels were higher than the anti-AQP4 antibody levels. This suggests that the two immune responses are independent rather than the one being byproduct/consequence of the other.

### Example 4 - Search for cross-reactivity between the two antibody groups (anti-AQP1 and anti-AQP4)

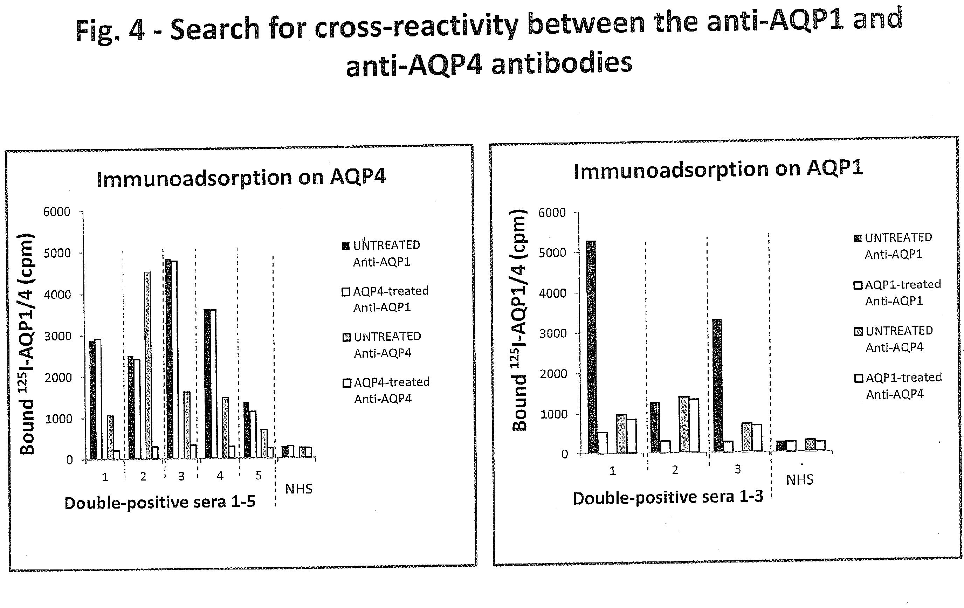

AQP1 and AQP4 belong to the same protein family and they have extensive amino acid sequence homology. The presence of sera with only anti-AQP1 or only anti-AQP4 antibodies shows that the two groups of antibodies (to AQP1 or AQP4) differ considerably and the binding to the two antigens is not due solely to cross-reactive antibodies. To test for any cross-reactive antibodies, we depleted selected sera from their antibodies binding to the one antigen and tested the depleted sera for binding to either antigen. Specifically, the test sera were treated with immobilized AQP1 or AQP4 (to immunoadsorb all antibodies binding to AQP1 or AQP4 respectively) and subsequently the treated sera were tested by RIPA using ¹²⁵I-labeled AQP1 and AQP4. Fig. 4 shows that the immobilized AQP1 practically completely eliminated the anti-AQP1 antibodies but did not reduce the anti-AQP4 values, and the reverse. Therefore no significant antibody cross-reactivity could be detected in the tested sera.

### Example 5 - Verification of the anti-AQP1 antibodies in low titer sera by the two-step assay

In order to easily screen the several hundreds of sera for anti-AQP1 antibodies, in the experiments described above, we used the regular RIPA instead of the more sensitive two-step RIPA we developed recently as described in PCT/IB2011/001014. The two-step RIPA was earlier used for the detection of anti-AQP4 antibodies in these sera. It is likely therefore that some sera with low levels of anti-AQP1 antibodies escaped detection. We here used the potency of the two-step RIPA for the verification of the positivity of some low positive sera, as detected with the regular RIPA. Fig. 5 confirms the positivity of some low positive (no. 1,3,4,5,7,8) or ambiguous sera (no. 2,6,9), whereas the Δcpm value of two "high cpm" NHS (the second had the highest precipitated cpm among the 100 tested NHS) did not increase suggesting that their above the average values are probably due to non-specific factors (e.g. possibly binding to a minor impurity in the AQP1 preparation) rather than to the presence of anti-AQP1 autoantibodies. Also one ambiguous serum (no 10) remained ambiguous by the two-step RIPA suggesting that it may not be really positive.

### Example 6 - Biochemical characterization of the autoantibody-target of the anti-AQP1 sera

To further biochemically characterize the target of the anti-AQP1 sera, using three anti-AQP1 positive sera we immunoprecipitated the antibody-antigen complexes and identified the bound antigen by PAGE followed by Western blotting. Specifically, the sera were incubated with biotinilated AQP1 followed by precipitation with Sepharose-protein A beads. The AQP1 indirectly bound on the beads (through the bound autoantibodies) was released, run on PAGE and revealed by Western blot using HRP-streptavidin and its substrate. Semi-quantitation of the precipitated AQP1 was achieved by comparison of the intensity of the bands by those of parallel analysis of specific quantities of biotinilated AQP1 subjected directly to PAGE and Western blot analysis.

Fig 6 shows that the antigen has the apparent molecular weight of the AQP1-GST fusion protein (∼55KD) and of its dimer (apparently due to GST dimerization), and that the serum antibodies (especially those of serum 1) bound to at least a large fraction of the used protein (by comparison with the intensity of the marker AQP1 bands) thus excluding the possibility that their antigen is an impurity in the AQP1 preparation with similar MW with AQP1.

### Example 7 - Identification of AQP1 antibody-binding pattern in mouse brain with IIF

Interestingly, in humans (but not in mice), AQP1 has similar cell type distribution with AQP4 in astrocytes (Satoh, Tabunoki et al. 2007; Moftakhar, Lynch et al. 2010), However, autoantibodies to AQP1 may not be detected with the usual IIF assay on mouse tissues used for NMO-IgG antibodies because a. this assay, in order to reduce background, usually involves treatment of the test serum with guinea pig liver powder, which incidentally contains AQP1 (Talbot, Garrett et al. 2003) and therefore any autoantibodies to AQP1 in the test serum would be eliminated and not detected; and b. even if this problem is overcome, mouse astrocytes do not express AQP1.

Elimination of the liver powder step is not preferred when using of intact sera since it would result in relatively high background. However, isolation of anti-AQP1 and anti-AQP4 antibodies by affinity chromatography may allow for omission of the liver powder step.

We isolated anti-AQP1 antibodies from sera from AQP1+ patients (and from AQP4+ and NHS, as negative controls) by affinity chromatography using immobilized AQP1. With samples purified from sera positive for AQP1 antibodies we detected a new specific pattern corresponding to AQP1 expression in the choroid plexus in the mouse brain. The corresponding samples "purified" from AQP4+ sera and from NHS did not result in significant staining. When whole untreated sera with anti-AQP1 antibodies were used (without liver powder treatment) a similar staining pattern was observed, but the background (by the use of NHS) was relatively high.

### Example 8 - Clinical/MRI and cytological findings in patients positive for anti-AQP1 antibodies

Most test sera were collected from patients during routine diagnosis for anti-AQP4 antibodies, with minimal clinical data available. We studied in detail the clinical data for 15 patients with only anti-AQP1 antibodies (anti-AQP1+). A retrospective analysis of MRI showed that 3 of them had NMO (longitudinally extensive transverse myelitis with optic neuritis), 7 had only longitudinally extensive transverse myelitis with brain lesions atypical for MS, one had only transverse myelitis, while 4 had MS (however with predominantly spinal cord lesions, the two which had longitudinally extensive transverse myelitis). Importantly, three of the 15 patients also had neoplasm (nephroma, non-Hodgkin lymphoma, or mammary cancer), suggesting anti-AQP1 autoantibodies as a possible novel paraneoplastic marker.

Our findings suggest the identification of a specific group of demyelinated disorders, distinct from classical MS and probably distinct from 'classical' NMO with only anti-AQP4 antibodies. AQP4 and AQP1 have different distribution in the CNS, with AQP4 expressed predominantly in the gray matter (brain and spinal cord) and AQP1 only in the white matter. Since patients positive only for AQP1 antibodies may have demyelination only in the white matter, we believe that the new marker, anti-AQP1 antibodies, is specific for a novel demyelinated autoimmune disorder, probably close relative to anti-AQP4 containing NMO ("classical NMO").

In addition to the CNS, AQP1 has been also detected in the peripheral nervous system (Ma, T. H., H. W. Gao, et al. (2011) Acta Pharmacol Sin 32(6): 711-715) and in non-neuronal tissues like red blood cells, liver, kidney and lungs. Therefore, autoantibodies to AQP1 may be involved and serve as markers in relevant diseases including idiopathic autoimmune hemolytic anemia (e.g. some patients improve with cortisone or even rituximab), autoimmune chronic obstructive pulmonary disease and peripheral neuropathies.

### Example 9 - Immunoadsorption of anti-AQP1 antibodies from patient sera - therapeutic indications

We then tested whether the autoantigen, AQP1, when immobilized could be used for the elimination of the anti-AQP1 autoantibodies from the patient's serum. AQP1 was immobilized on ELISA plates; to the plates was added anti-AQP1 positive serum and incubated. Then the treated serum was tested by RIPA to determine whether the serum was depleted from the autoantibodies. Fig. 7 shows that the serum was practically completely depleted of all anti-AQP1 autoantibodies. This experiment suggests that it is possible to use immobilized AQP1 or its fragments and mutants, to specifically eliminate the patient's antibodies from the circulation, during ex vivo immunoadsorption procedure for therapeutic purposes.

### Example 10 - Detection of antibodies to AQP-2, -5, -7 and -8 in sera from patients suspected for having NMO/NMOsd

We examined sera from 362 patients (described in Example 1), suspected for NMO, for the presence antibodies to AQP2, -5, -7, and -8. Antibodies to AQP5 were detected by the cell based assay procedure set out in Waters, P. et al. (2008). "Aquaporin-4 antibodies in neuromyelitis optica and longitudinally extensive transverse myelitis." Arch Neurol 65(7): 913-919. Antibodies to AQP2, -7, and -8 were detected by plain RIPA (not two step RIPA) described in Example 1.

Table 1 shows that antibodies to most of these AQPs were detected in some test sera, though with lower frequency than anti-AQP1/4 antibodies, but not in sera from healthy controls. Most positive sera had antibodies to only one AQP (11 sera), but 5 sera had antibodies to more than one AQP: One serum was positive for three AQPs (AQPs 1, 2 and 8), two sera were positive for AQPs 1 and 8, one for AQPs 4 and 8, and one serum was positive for AQPs 1 and 5.

**Table 1. Detection of antibodies to AQP-2, -5, -7 and -8 in sera from patients suspected for having NMO-like symptoms**

| | **Detection of antibodies to:** | | | |
|---|---|---|---|---|
| | AQP2 | AQP5 | AQP7 | AQP8 |
| **Tested** | Positive/total tested | | | |
| **sera from:** | | | | |
| Patients suspected for NMO-like symptoms | 3/86 (3.5%) | 2/22 (9%) | 4/65 (6.2%) | 8/138 (5.8%) |
| Healthy controls | 0/28 | 0/9 | 0/12 | 0/55 |

Various modifications and variations of the described methods and system of the present invention will be apparent to those skilled in the art without departing from the scope I of the present invention. Although the present invention has been described In connection with specific preferred embodiments, it should be understood that the invention as claimed should not be unduly limited to such specific embodiments. Indeed, various modifications of the described modes for carrying out the invention which are obvious to those skilled in biochemistry, immunology and biotechnology or related fields are intended to be within the scope of the following claims.

## Claims

1. A method of diagnosis or prognosis of an autoimmune disease associated with the formation of demyelinated lesions of the central nervous system (CNS) in a subject wherein the method comprises detecting aquaporin-1 (AQP1) autoantibodies in a sample obtained from said subject.

2. A method of monitoring the progress of an autoimmune disease associated with the formation of demyelinated lesions of the CNS in a subject comprising the steps of:
(i) providing a first sample obtained from the subject,
(ii) detecting AQP1 autoantibodies in said first sample,
(iii) providing a second sample from the subject wherein said second sample is obtained from the subject after said first sample,
(iv) comparing the level of the AQP1 autoantibodies in the second sample with the corresponding level of AQP1 autoantibodies in the first sample, wherein an increase in level of the AQP1 autoantibodies in the second sample relative to the first sample indicates disease progression.

3. A method according to claim 2 wherein the second sample is obtained from the subject at least 10, 20, 50, 100, 200 or 300 days after the first sample, and/or wherein the method is for monitoring disease progression in response to treatment of said disease.

4. A method according to any preceding claim wherein the AQP1 autoantibodies are detected by contacting said sample with AQP1 polypeptide, or a cell or tissue comprising said polypeptide, and detecting the presence or absence of binding of said AQP1 autoantibodies to said AQP1 polypeptide, cell or tissue.

5. A method according to any preceding claim wherein the sample is nervous tissue, blood, blood cells, blood plasma, blood serum or cerebrospinal fluid.

6. The method according to any preceding claim wherein the AQP1 autoantibodies are detected by an immuoassay.

7. A method according to claim 6 wherein the immunoassay is selected from enzyme-linked immunosorbent assay (ELISA), radioimmunoassay (RIA), immunoprecipitation assay, immunofluorescence assay (IFA), indirect immunofluorescence (IIF) assay enzyme linked assay (EIA), luminescence immunoassay (LIA), Western Blot assay (WB) and cell based assay (CBA).

8. An AQP1 polypeptide or a cell or tissue comprising said polypeptide, for use in diagnosing an autoimmune disease associated with the formation of demyelinated lesions of the CNS.

9. A method according to any preceding claim wherein the method further comprises detecting aquaporin-4 (AQP4) autoantibodies.

10. A method according to any of claims 1 to 7 or 9, or an AQP1 polypeptide for use according to claim 8 wherein the autoimmune disease is Neuromyelitis Optica (NMO).

11. An AQP1 polypeptide for use in treating a disease **characterised by** the presence of, or elevated levels of, AQP1 autoantibody wherein said disease is an autoimmune disease associated with the formation of demyelinated lesions of the CNS.

12. An AQP1 polypeptide according to claim 11 wherein the AQP1 polypeptide is incorporated into a particle such as a nanoparticle or a liposome.

## Patentansprüche

1. Verfahren zum Diagnostizieren oder Prognostizieren einer mit der Ausbildung von demyelinisierten Läsionen des zentralen Nervensystems (ZNS) verknüpften Autoimmunerkrankung in einem Subjekt, wobei das Verfahren das Nachweisen von Aquaporin-1(AQP1)-Autoantikörpern in einer von dem Subjekt erhaltenen Probe umfasst.

2. Verfahren zum Überwachen des Fortschreitens einer mit der Ausbildung von demyelinisierten Läsionen des ZNS verknüpften Autoimmunerkrankung in einem Subjekt, die folgenden Schritte umfassend:
(i) Bereitstellen einer ersten von dem Subjekt erhaltenen Probe,
(ii) Nachweisen von AQP1-Autoantikörpern in der ersten Probe,
(iii) Bereitstellen einer zweiten Probe von dem Subjekt, wobei die zweite Probe nach der ersten Probe von dem Subjekt erhalten wird,
(iv) Vergleichen des Spiegels der AQP1-Autoantikörper in der zweiten Probe mit dem entsprechenden Spiegel von AQP1-Autoantikörpern in der ersten Probe, wobei ein Anstieg des Spiegels der AQP1-Autoantikörper in der zweiten Probe im Vergleich zu der ersten Probe ein Fortschreiten der Erkrankung andeutet.

3. Verfahren nach Anspruch 2, wobei die zweite Probe mindestens 10, 20, 50, 100, 200 oder 300 Tage nach der ersten Probe von dem Subjekt erhalten wird und/oder wobei das Verfahren zum Überwachen des Fortschreitens der Erkrankung als Reaktion auf eine Behandlung der Erkrankung dient.

4. Verfahren nach einem der vorhergehenden Ansprüche, wobei die AQP1-Autoantikörper nachgewiesen werden, indem die Probe mit AQP1-Polypeptid oder mit einer Zelle oder mit Gewebe, die/das das Polypeptid umfasst, in Berührung gebracht wird und das Vorliegen oder das Fehlen einer Bindung der AQP1-Autoantikörper an das AQP1-Polypeptid, die Zelle oder das Gewebe nachgewiesen wird.

5. Verfahren nach einem der vorhergehenden Ansprüche, wobei die Probe Nervengewebe, Blut, Blutzellen, Blutplasma, Blutserum oder Zerebrospinalflüssigkeit ist.

6. Verfahren nach einem der vorhergehenden Ansprüche, wobei die AQP1-Autoantikörper durch einen Immunassay nachgewiesen werden.

7. Verfahren nach Anspruch 6, wobei der Immunassay ausgewählt ist aus Enzyme-linked Immunosorbent Assay (ELISA), Radioimmunassay (RIA), Immunpräzipitationsassay, Immunofluoreszenzassay (IFA), indirektem Immunfluoreszenz(IIF)-Assay, enzymgekoppeltem Assay (EIA), Lumineszenz-Immunassay (LIA), Western-Blot-Assay (WB) und zellbasiertem Assay (CBA).

8. AQP1-Polypeptid oder Zelle oder Gewebe, die/das das Polypeptid umfasst, für den Einsatz beim Diagnostizieren einer mit der Ausbildung von demyelinisierten Läsionen des ZNS verknüpften Autoimmunerkrankung.

9. Verfahren nach einem der vorhergehenden Ansprüche, wobei das Verfahren ferner das Nachweisen von Aquaporin-4(AQP4)-Autoantikörpern umfasst.

10. Verfahren nach einem der Ansprüche 1 bis 7 oder 9 oder AQP1-Polypeptid für den Einsatz nach Anspruch 8, wobei die Autoimmunerkrankung Neuromyelitis optica (NMO) ist.

11. AQP1-Polypeptid für den Einsatz beim Behandeln einer Erkrankung, **gekennzeichnet durch** das Vorliegen von oder durch erhöhte Spiegel von AQP1-Autoantikörper, wobei die Erkrankung eine mit der Ausbildung von demyelinisierten Läsionen des ZNS verknüpfte Autoimmunerkrankung ist.

12. AQP1-Polypeptid nach Anspruch 11, wobei das AQP1-Polypeptid in ein Partikel, wie etwa ein Nanopartikel oder ein Liposom, eingebunden ist.

## Revendications

1. Procédé de diagnostic ou de pronostic d'une maladie auto-immune associée à la formation de lésions démyélinisées du système nerveux central (SNC) chez un sujet, le procédé comprenant la détection d'autoanticorps d'aquaporine-1 (AQP1) dans un échantillon obtenu dudit sujet.

2. Procédé de surveillance de la progression d'une maladie auto-immune associée à la formation de lésions démyélinisées du SNC chez un sujet comprenant les étapes de :
(i) mise à disposition d'un premier échantillon obtenu du sujet,
(ii) détection d'autoanticorps d'AQP1 dans ledit premier échantillon,
(iii) mise à disposition d'un deuxième échantillon chez le sujet, ledit deuxième échantillon étant obtenu du sujet après ledit premier échantillon,
(iv) comparaison du niveau d'autoanticorps d'AQP1 dans le deuxième échantillon avec le niveau correspondant d'autoanticorps d'AQP1 dans le premier échantillon, une augmentation du niveau des autoanticorps d'AQP1 dans le 2^{e} échantillon par rapport au premier échantillon indiquant une progression de la maladie.

3. Procédé selon la revendication 2, le deuxième échantillon étant obtenu du sujet au moins 10, 20, 50, 100, 200 ou 300 jours après le premier échantillon, et/ou le procédé étant destiné à la surveillance de la progression d'une maladie en réponse à un traitement de ladite maladie.

4. Procédé selon l'une quelconque des revendications précédentes, les autoanticorps d'AQP1 étant détectés par mise en contact dudit échantillon avec le polypeptide d'AQP1, ou une cellule ou un tissu comprenant ledit polypeptide, et détection de la présence ou de l'absence de liaison desdits autoanticorps d'AQP1 audit polypeptide, à ladite cellule ou audit tissu d'AQP1.

5. Procédé selon une quelconque revendication précédente, l'échantillon étant un tissu nerveux, du sang, des cellules sanguines, un plasma sanguin, un sérum sanguin ou un fluide cérébrospinal.

6. Procédé selon une quelconque revendication précédente, les autoanticorps d'AQP1 étant détectés par un dosage immunologique.

7. Procédé selon la revendication 6, le dosage immunologique étant choisi parmi un dosage immunoenzymatique (ELISA), un dosage radioimmunologique (RIA), un dosage par immunoprécipitation, un dosage par immunofluorescence (IFA), un dosage par immunofluorescence indirecte (IIF), un dosage enzymatique (EIA), un dosage immunologique par luminescence (LIA), un dosage Western Blot (WB) et un dosage cellulaire (CBA).

8. Polypeptide d'AQP1 ou cellule ou tissu comprenant ledit polypeptide, pour une utilisation dans un diagnostic d'une maladie auto-immune associée à la formation de lésions démyélinisées du SNC.

9. Procédé selon une quelconque revendication précédente, le procédé comprenant en outre la détection d'autoanticorps d'aquaporine-4 (AQP4).

10. Procédé selon l'une quelconque des revendications 1 à 7 ou 9, ou polypeptide d'APQ1 pour une utilisation selon la revendication 8, la maladie auto-immune étant la neuromyélite optique (NMO).

11. Polypeptide d'AQP1 pour une utilisation dans un traitement d'une maladie **caractérisée par** la présence, ou de niveaux élevés, d'autoanticorps d'AQP1, ladite maladie étant une maladie auto-immune associée à la formation de lésions démyélinisées du SNC.

12. Polypeptide d'AQP1 selon la revendication 11, le polypeptide d'AQP1 étant incorporé dans une particule telle qu'une nanoparticule ou un liposome.


### SEQUENCE LISTING

<110> Tzartos, Socrates
<120> Biomarker
<130> P045457PCT
<150> GR 20110100593
   <151> 2011-10-17
<160> 10
<170> PatentIn version 3.5
<210> 1
   <211> 850
   <212> DNA
   <213> Homo sapiens
<400> 1
   gtacaaaaaa gcaggctcca ccatggccag cgagttcaag aagaagctct tctggagggc      60
   agtggtggcc gagttcctgg ccacgaccct ctttgtcttc atcagcatcg gttctgccct     120
   gggcttcaaa tacccggtgg ggaacaacca gacgacggtc caggacaacg tgaaggtgtc     180
   gctggccttc gggctgagca tcgccacgct ggcgcagagt gtgggccaca tcagcggcgc     240
   ccacctcaac ccggctgtca cactggggct gctgctcagc tgccagatca gcatcttccg     300
   tgccctcatg tacatcatcg cccagtgcgt gggggccatc gtcgccaccg ccatcctctc     360
   aggcatcacc tcctccctga ctgggaactc gcttggccgc aatgacctgg ctgatggtgt     420
   gaactcgggc cagggcctgg gcatcgagat catcgggacc ctccagctgg tgctatgcgt     480
   gctggctact accgaccgga ggcgccgtga ccttggtggc tcagcccccc ttgccatcgg     540
   cctctctgta gcccttggac acctcctggc tattgactac actggctgtg ggattaaccc     600
   tgctcggtcc tttggctccg cggtgatcac acacaacttc agcaaccact ggattttctg     660
   ggtggggcca ttcatcgggg gagccctggc tgtactcatc tacgacttca tcctggcccc     720
   acgcagcagt gacctcacag accgcgtgaa ggtgtggacc agcggccagg tggaggagta     780
   tgacctggat gccgacgaca tcaactccag ggtggagatg aagcccaaat tggacccagc     840
   tttcttgtac                                                            850
<210> 2
   <211> 269
   <212> PRT
   <213> Homo sapiens
<400> 2
   Met Ala Ser Glu Phe Lys Lys Lys Leu Phe Trp Arg Ala Val Val Ala
   1               5                   10                  15
   Glu Phe Leu Ala Thr Thr Leu Phe Val Phe Ile Ser Ile Gly Ser Ala
               20                  25                  30
   Leu Gly Phe Lys Tyr Pro Val Gly Asn Asn Gln Thr Ala Val Gln Asp
           35                  40                  45
   Asn Val Lys Val Ser Leu Ala Phe Gly Leu Ser Ile Ala Thr Leu Ala
       50                  55                  60
   Gln Ser Val Gly His Ile Ser Gly Ala His Leu Asn Pro Ala Val Thr
   65                  70                  75                  80
   Leu Gly Leu Leu Leu Ser Cys Gln Ile Ser Ile Phe Arg Ala Leu Met
                   85                  90                  95
   Tyr Ile Ile Ala Gln Cys Val Gly Ala Ile Val Ala Thr Ala Ile Leu
               100                 105                 110
   Ser Gly Ile Thr Ser Ser Leu Thr Gly Asn Ser Leu Gly Arg Asn Asp
           115                 120                 125
   Leu Ala Asp Gly Val Asn Ser Gly Gln Gly Leu Gly Ile Glu Ile Ile
       130                 135                 140
   Gly Thr Leu Gln Leu Val Leu Cys Val Leu Ala Thr Thr Asp Arg Arg
   145                 150                 155                 160
   Arg Arg Asp Leu Gly Gly Ser Ala Pro Leu Ala Ile Gly Leu Ser Val
                   165                 170                 175
   Ala Leu Gly His Leu Leu Ala Ile Asp Tyr Thr Gly Cys Gly Ile Asn
               180                 185                 190
   Pro Ala Arg Ser Phe Gly Ser Ala Val Ile Thr His Asn Phe Ser Asn
           195                 200                 205
   His Trp Ile Phe Trp Val Gly Pro Phe Ile Gly Gly Ala Leu Ala Val
       210                 215                 220
   Leu Ile Tyr Asp Phe Ile Leu Ala Pro Arg Ser Ser Asp Leu Thr Asp
   225                 230                 235                 240
   Arg Val Lys Val Trp Thr Ser Gly Gln Val Glu Glu Tyr Asp Leu Asp
                   245                 250                 255
   Ala Asp Asp Ile Asn Ser Arg Val Glu Met Lys Pro Lys
               260                 265
<210> 3
   <211> 995
   <212> DNA
   <213> Homo sapiens
<400> 3
   agtgcgagag cgagtgcccg gagcatcctg gccctgagac agctgggcca gccccgcagg      60
   gctctgcagc atgtgggagc tccgctccat agccttctcc agggctgtgt tcgcagagtt     120
   cctggccaca ctcctcttcg tcttctttgg cctcggctct gccctcaact ggccacaggc     180
   cctgccctct gtgctacaga ttgccatggc gtttggcttg ggtattggca ccctggtaca     240
   ggctctgggc cacataagcg gggcccacat caaccctgcc gtgactgtgg cctgcctggt     300
   gggctgccac gtctccgttc tccgagccgc cttctacgtg gctgcccagc tgctgggggc     360
   tgtggccgga gccgctctgc tccatgagat cacgccagca gacatccgcg gggacctggc     420
   tgtcaatgct gtgagtagcc acaactttgc tcttctctgt ccccagctca gcaacagcac     480
   gacggctggc caggcggtga ctgtggagct cttcctgaca ctgcagctgg tgctctgcat     540
   cttcgcctcc accgatgagc gccgcggaga gaacccgggc acccctgctc tctccatagg     600
   cttctccgtg gccctgggcc acctccttgg ggtaggtcat ggccatgccc tcctcccact     660
   gcagatccat tacaccggct gctctatgaa tcctgcccgc tccctggctc cagctgtcgt     720
   cactggcaaa tttgatgacc actgggtaat ggctgaatcc gctcgccccc aggtcttctg     780
   gatcggaccc ctggtgggcg ccatcctggg ctccctcctc tacaactacg tgctgtttcc     840
   gccagccaag agcctgtcgg agcgcctggc agtgctgaag ggcctggagc cggacaccga     900
   ttgggaggag cgcgaggtgc gacggcggca gtcggtggag ctgcactcgc cgcagagcct     960
   gccacggggt accaaggcct gagggccgcc agcgg                                995
<210> 4
   <211> 271
   <212> PRT
   <213> Homo sapiens
<400> 4
<210> 5
   <211> 848
   <212> DNA
   <213> Homo sapiens
<400> 5
   gggtccgtgg gtctgtcctc tgtggggtgg ggggcaggtc ttcaaggtct gggctcagcg        60
   ccctgactcg tgccctgtct ccaccaggtt ttctgggtag ggcccatcgt gggggcggtc       120
   ctggctgcca tcctttactt ctacctgctc ttccccaact ccctgagcct gagtgagcgt      180
   gtggccatca tcaaaggcac gtatgagcct gacgaggact gggaggagca gcgggaagag      240
   cggaagaaga ccatggagct gaccacccgc tgaccagtgt caggcagggg ccagcccctc      300
   agcccctgag ccaaggggga aaagaagaaa aagatcctaa cacaagcttc ctttttgcac      360
   aaccggtcct cttggctgag gaggaggagc tggtcaccct ggctgcacag ttagagaggg      420
   gagaaggaac ccatgatggg actcctgggg taggggccag gggctggggt ctgctgggga      480
   caggtctctc tgggacagac ctcagagatt ctcaatgcag tgccaagctc acaggctgca      540
   agggccaggc cagaaaaggg tgggcctgca gcctgcaccc cccaccttcc ccaacccttc      600
   ctcaagagct gaagggatcc cagaccctag gtgggcagag gcagaccctc cccagagctc      660
   cttaggaaga agacagactg gttcattgaa tgccgcctta tttatttctg gtgaggatgc      720
   atgcgtgggg ctgctggtct ttagagtggg ggctacccaa taaatcactg atactcacat      780
   tccgcctctg tctctcctca gagtgccttg agacactctg ccccattgcc tctcctcttt      840
   gtcatccc                                                                848
<210> 6
   <211> 265
   <212> PRT
   <213> Homo sapiens
<400> 6
<210> 7
   <211> 1258
   <212> DNA
   <213> Homo sapiens
<400> 7
   ggctctggac tggggacaca gggatagctg agccccagct gggggtggaa gctgagccag      60
   ggacagtcac ggaggaacaa gatcaagatg cgctgtaact gagaagcccc caaggcggag     120
   gctgagaatc agagacattt cagcagacat ctacaaatct gaaagacaaa acatggttca     180
   agcatccggg cacaggcggt ccacccgtgg ctccaaaatg gtctcctggt ccgtgatagc     240
   aaagatccag gaaatactgc agaggaagat ggtgcgagag ttcctggccg agttcatgag     300
   cacatatgtc atgatggtat tcggccttgg ttccgtggcc catatggttc taaataaaaa     360
   atatgggagc taccttggtg tcaacttggg ttttggcttc ggagtcacca tgggagtgca     420
   cgtggcaggc cgcatctctg gagcccacat gaacgcagct gtgacctttg ctaactgtgc     480
   gctgggccgc gtgccctgga ggaagtttcc ggtctatgtg ctggggcagt tcctgggctc     540
   cttcctggcg gctgccacca tctacagtct cttctacacg gccattctcc acttttcggg     600
   tggacagctg atggtgaccg gtcccgtcgc tacagctggc atttttgcca cctaccttcc     660
   tgatcacatg acattgtggc ggggcttcct gaatgaggcg tggctgaccg ggatgctcca     720
   gctgtgtctc ttcgccatca cggaccagga gaacaaccca gcactgccag gaacagaggc     780
   gctggtgata ggcatcctcg tggtcatcat cggggtgtcc cttggcatga acacaggata     840
   tgccatcaac ccgtcccggg acctgccccc ccgcatcttc accttcattg ctggttgggg     900
   caaacaggtc ttcagcaatg gggagaactg gtggtgggtg ccagtggtgg caccacttct     960
   gggtgcctat ctaggtggca tcatctacct ggtcttcatt ggctccacca tcccacggga    1020
   gcccctgaaa ttggaggatt ctgtggcgta tgaagaccac gggataaccg tattgcccaa    1080
   gatgggatct catgaaccca cgatctctcc cctcaccccc gtctctgtga gccctgccaa    1140
   cagatcttca gtccaccctg ccccaccctt acatgaatcc atggccctag agcacttcta    1200
   agcagagatt atttgtgatc ccatccattc cccaataaag caaggcttgt ccgacaaa      1258
<210> 8
   <211> 342
   <212> PRT
   <213> Homo sapiens
<400> 8
   Met Val Gln Ala Ser Gly His Arg Arg Ser Thr Arg Gly Ser Lys Met
   1               5                   10                  15
   Val Ser Trp Ser Val Ile Ala Lys Ile Gln Glu Ile Leu Gln Arg Lys
               20                  25                  30
   Met Val Arg Glu Phe Leu Ala Glu Phe Met Ser Thr Tyr Val Met Met
           35                  40                  45
   Val Phe Gly Leu Gly Ser Val Ala His Met Val Leu Asn Lys Lys Tyr
       50                  55                  60
   Gly Ser Tyr Leu Gly Val Asn Leu Gly Phe Gly Phe Gly Val Thr Met
   65                  70                  75                  80
   Gly Val His Val Ala Gly Arg Ile Ser Gly Ala His Met Asn Ala Ala
                   85                  90                  95
   Val Thr Phe Ala Asn Cys Ala Leu Gly Arg Val Pro Trp Arg Lys Phe
               100                 105                 110
   Pro Val Tyr Val Leu Gly Gln Phe Leu Gly Ser Phe Leu Ala Ala Ala
           115                 120                 125
   Thr Ile Tyr Ser Leu Phe Tyr Thr Ala Ile Leu His Phe Ser Gly Gly
       130                 135                 140
   Gln Leu Met Val Thr Gly Pro Val Ala Thr Ala Gly Ile Phe Ala Thr
   145                 150                 155                 160
   Tyr Leu Pro Asp His Met Thr Leu Trp Arg Gly Phe Leu Asn Glu Ala
                   165                 170                 175
   Trp Leu Thr Gly Met Leu Gln Leu Cys Leu Phe Ala Ile Thr Asp Gln
               180                 185                 190
   Glu Asn Asn Pro Ala Leu Pro Gly Thr Glu Ala Leu Val Ile Gly Ile
           195                 200                 205
   Leu Val Val Ile Ile Gly Val Ser Leu Gly Met Asn Thr Gly Tyr Ala
       210                 215                 220
   Ile Asn Pro Ser Arg Asp Leu Pro Pro Arg Ile Phe Thr Phe Ile Ala
   225                 230                 235                 240
   Gly Trp Gly Lys Gln Val Phe Ser Asn Gly Glu Asn Trp Trp Trp Val
                   245                 250                 255
   Pro Val Val Ala Pro Leu Leu Gly Ala Tyr Leu Gly Gly Ile Ile Tyr
               260                 265                 270
   Leu Val Phe Ile Gly Ser Thr Ile Pro Arg Glu Pro Leu Lys Leu Glu
           275                 280                 285
   Asp Ser Val Ala Tyr Glu Asp His Gly Ile Thr Val Leu Pro Lys Met
       290                 295                 300
   Gly Ser His Glu Pro Thr Ile Ser Pro Leu Thr Pro Val Ser Val Ser
   305                 310                 315                 320
   Pro Ala Asn Arg Ser Ser Val His Pro Ala Pro Pro Leu His Glu Ser
                   325                 330                 335
   Met Ala Leu Glu His Phe
               340
<210> 9
   <211> 1309
   <212> DNA
   <213> Homo sapiens
<400> 9
   taggagataa gagtatcttg cacagcaggt gcaggtttcc cagcagctca ggcaagagtc      60
   cgatgtttgt gccatctgat cctgatgtct ggagagatag ccatgtgtga gcctgaattt     120
   ggcaatgaca aggccaggga gccgagcgtg ggtggcaggt ggcgagtgtc ctggtacgaa     180
   cggtttgtgc agccatgtct ggtcgaactg ctgggctctg ctctcttcat cttcatcggg     240
   tgcctgtcgg tcattgagaa tgggacggac actgggctgc tgcagccggc cctggcccac     300
   gggctggctt tggggctcgt gattgccacg ctggggaata tcagtggtgg acacttcaac     360
   cctgcggtgt ccctggcagc catgctgatc ggaggcctca acctggtgat gctcctcccg     420
   tactgggtct cacagctgct cggggggatg ctcggggctg ccttggccaa ggtggtgagt    480
   cctgaggaga ggttctggaa tgcatctggg gcggcctttg tgacagtcca ggagcagggg     540
   caggtggcag gggcgttggt ggcagagatc atcctgacga cgctgctggc cctggctgta     600
   tgcatgggtg ccatcaatga gaagacaaag ggccctctgg ccccgttctc catcggcttt     660
   gccgtcaccg tggatatcct ggctgggggc cctgtgtctg gaggctgcat gaatcccgcc     720
   cgtgcttttg gacctgcggt ggtggccaac cactggaact tccactggat ctactggctg     780
   ggcccactcc tggctggcct gcttgttgga ctgctcatta ggtgcttcat tggagatggg     840
   aagacccgcc tcatcctgaa ggctcggtga gcagagctcg tgggattcct gctgctccag     900
   gtgtcctcag ctcacctgtc ccagactgag gacaggggag ttcctgcatt tcctgccagg     960
   gcagaggccc agaggagcga ccccctgctt ccactgcttg ggcctgcttt ctcagataga   1020
   ctgactgctg aggaggctct aggttcttgg aattcctttg tgctcatcag agaccccagc    1080
   ctggggaaca cgctgcccgc actgcccaga gagcagtgca aacaccacaa cacgagcgtg    1140
   tttcttgaga ggaatgtccc cgagttggac aaggaggctg tttctgcaca tcagctcatt    1200
   tcccgcaccc catttcttgc ttgattgctt tgttgggggc ctggccactt ccttgcttct    1260
   caagctgaca attctcactt tgcaataaat agtccagtgt ttccttcat                1309
<210> 10
   <211> 261
   <212> PRT
   <213> Homo sapiens
<400> 10
   Met Ser Gly Glu Ile Ala Met Cys Glu Pro Glu Phe Gly Asn Asp Lys
   1               5                   10                  15
   Ala Arg Glu Pro Ser Val Gly Gly Arg Trp Arg Val Ser Trp Tyr Glu
               20                  25                  30
   Arg Phe Val Gln Pro Cys Leu Val Glu Leu Leu Gly Ser Ala Leu Phe
           35                  40                  45
   Ile Phe Ile Gly Cys Leu Ser Val Ile Glu Asn Gly Thr Asp Thr Gly
       50                  55                  60
   Leu Leu Gln Pro Ala Leu Ala His Gly Leu Ala Leu Gly Leu Val Ile
   65                  70                  75                  80
   Ala Thr Leu Gly Asn Ile Ser Gly Gly His Phe Asn Pro Ala Val Ser
                   85                  90                  95
   Leu Ala Ala Met Leu Ile Gly Gly Leu Asn Leu Val Met Leu Leu Pro
               100                 105                 110
   Tyr Trp Val Ser Gln Leu Leu Gly Gly Met Leu Gly Ala Ala Leu Ala
           115                 120                 125
   Lys Ala Val Ser Pro Glu Glu Arg Phe Trp Asn Ala Ser Gly Ala Ala
       130                 135                 140
   Phe Val Thr Val Gln Glu Gln Gly Gln Val Ala Gly Ala Leu Val Ala
   145                 150                 155                 160
   Glu Ile Ile Leu Thr Thr Leu Leu Ala Leu Ala Val Cys Met Gly Ala
                   165                 170                 175
   Ile Asn Glu Lys Thr Lys Gly Pro Leu Ala Pro Phe Ser Ile Gly Phe
               180                 185                 190
   Ala Val Thr Val Asp Ile Leu Ala Gly Gly Pro Val Ser Gly Gly Cys
           195                 200                 205
   Met Asn Pro Ala Arg Ala Phe Gly Pro Ala Val Val Ala Asn His Trp
       210                 215                 220
   Asn Phe His Trp Ile Tyr Trp Leu Gly Pro Leu Leu Ala Gly Leu Leu
   225                 230                 235                 240
   Val Gly Leu Leu Ile Arg Cys Phe Ile Gly Asp Gly Lys Thr Arg Leu
                   245                 250                 255
   Ile Leu Lys Ala Arg
               260